# EUROPEAN PATENT SPECIFICATION

(11) **EP 1 290 442 B1**
(45) Date of publication and mention of the grant of the patent: **13.11.2013**
(21) Application number: 01938001.3
(22) Date of filing: 30.05.2001
(51) Int. Cl.: G01N 33/50, G01N 30/90

(54) **AN ASSAY METHOD FOR TESTING PLANT MATERIAL FOR EXPOSURE TO HERBICIDES USING BIOMARKERS**
EIN UNTERSUCHUNGSVERFAHREN ZUM TESTEN PFLANZLICHER MATERIALIEN AUF HERBIZIDE MITTELS BIOMARKERN
METHODE PERMETTANT D'ANALYSER UNE PLANTE EXPOSEE AUX HERBICIDES, A L'AIDE DE BIOMARQUEURS

(30) Priority: 30.05.2000 DK 200000874
(43) Date of publication of application: 12.03.2003
(73) Proprietor: Aarhus Universitet, 8000 Århus C (DK)
(72) Inventor: RAVN, Helle, Weber, DK-7451 Sunds (DK)
(74) Representative: Høiberg A/S
(86) International application number: PCT/DK2001/000377
(87) International publication number: WO 2001/092879

(56) References cited:
- EP-A- 0 832 557
- WO-A-00/28072
- US-A- 3 832 134
- US-A- 5 464 750
- US-A- 5 958 785
- STASIAK M A ET AL: "PHYSIOLOGICAL CHANGES INDUCED IN BIRCH SEEDLINGS BY SUBLETHAL APPLICATIONS OF GLYPHOSATE", CANADIAN JOURNAL OF FOREST RESEARCH, NATIONAL RESEARCH COUNCIL OF CANADA, OTTAWA, CA, vol. 22, no. 6, 1 January 1992 (1992-01-01), pages 812-817, XP000915743, ISSN: 0045-5067

## Description

### Technical field of the invention

The present invention concerns the detection of biomarkers in plant material. More specifically the invention rotates to a method of testing whether a plant has been exposed to herbicide exposure. The inven-tion further relates to the use of such a method of testing.

### Background of the invention

The use of biomarkers in the field of environmental and/or health assessment is getting increasing attention. Biomarkers may be defined as a biological response that can be related to an exposure to, or toxic effect of, environmental compound(s), such as chemicals. Biomarkers may provide evidence about the linkage between the exposure to toxic chemicals and the ecologically and/or health related relevant effects.

Biomarkers may give an impression of whether a living organism has been exposed to certain compounds, such as pesticides. Many insecticides, and to a lesser degree fungicides, are acutely toxic to animals (Thomson, 1987 & 1989).

Pesticides are vastly used in developed countries where modern intensive agriculture requires that productivity is optimised. The use of herbicides are wide-spread as well. The most prominent effect of herbicides is through adverse lethal effects on plants. The lethal effects are manifested by the change in the plant species composition and diversity, and by the alteration of the heterogenety of wildlife habitats. The effects of herbicides may also be on a cellular and/or molecular level, for example by the modification of plant development, growth and morphology. Ishikura et al., 1986 describe how the exposure of the herbicide glyphosate to plant cell cultures influences the some of the biochemical pathways in the cells, and thus changes the biochemical balance when compared to plant cells not being exposed to herbicide. Other research groups have focused on examining the effects of exposure of plants to herbicides. Evidence that herbicides influence the metabolism of phenol compounds in soybean tissue are disclosed by Hoagland et al., 1979. Also, the changes in the composition of secondary metabolites in plants exposed to glyphosate in described by Berlin & Witte, 1981 who studied the effect of glyphosate on shikimic acid accumulation in tobacco cell cultures with high and low yields of cinnamoyl putrescines. In the presence of glyphosate the levels of free shikimic acid were increased more than 300-fold by two cell lines. Lydon & Duke, 1989 reported that by virtue of the glyphosate inhibition of 5-enolpyruvyl shikimate-3-phospate (EPSP) synthase, the synthesis of all cinnamate derivatives were blocked in plant cell cultures. This lead to an accumulation of high levels of shikimate, benzoic acid, and benzoic acid derivatives.

Another research group showed that water stress changes the concentration of proline and soluble sugars in nodulated alfalfa, *Medicago sativa,* plants (Irigoyen et al., 1992).

In US 5,464,750 an assay and kit for the detection of Heat Shock Proteins (HSP) in organisms, such as invertebrates and plants, which have been subjected to sublethal doses of pollutants. The physiological stress of marine invertebrates was determined by correlating the level of HSP's with the degree of growth and reproduction success.

In another patent US 5,958,785 ethyl glucuronid was used as a biomarker for alcoholism in a human being and was detected by using Thin Layer Chromatography.

WO 00/28072 concerns a immunoassay for the determination of one or more oxidative damaged proteins in organisms, such as plants and animals. The quantity of biomarker was compared with a control value.

In 1992, Stasiak *et al*. studied sub-lethal effects of glyphosate on white birch seedlings grown outdoors and under controlled-environment conditions. Leaf fluorescence, photosynthetic pigments, ethylene, and changes in the shikimic acid pathway were used as indicators of these effects. They found physiological changes induced in birch (*Betula papyrifera* Marsh.) by sublethal applications of glyphosate. Chlorophyll a and b levels decreased within 4 days of application and carotenoid and gallic acid content increased, shikimic acid levels increased 10-fold within 24h application even at 1 % of field rate, and were still elevated in the spring of the following year.

It has been established that the sensitivity of plant species to herbicides varies. Boutin et al., 2001 (in prep.) describe the observation of the difference in sensitivity of different plant species exposed to different herbicides using visual effect signs.

Plant species were exposed to herbicide concentrations of 1% of recommended dose. The plants expressed biomarkers that were different with respect to the visual effect.

Clearly, the detection of pesticide exposure to living organisms, in particular plants is of interest not only to society as a whole in terms of financial concerns but also to the farming industry and to individual consumers. Prior art tests of determining the effects on for example plants after exposure to stress, such as pesticides have proven to be expensive, labour intensive having a low sensitivity and unable to detect effects on plants exposed to pesticides before visual signs appeared on the plant.

However, the present invention discloses a new and improved highly sensitive method of testing the effects on plant material having been exposed to a herbicide by taking advantage of the change in the composition of biomarkers in the material from said plant. Particularly, the present invention relates to a method of testing the non-visual as well as the visual effects on plants exposed to herbicides.

### Summary

The invention relates to a method of testing the composition of biomarkers as a tool to estimate whether a plant has been exposed to a herbicide as formulated in the appended claims.

In one aspect the invention relates to a method for testing whether material from a a specific plant has been exposed to a specific herbicide, comprising the steps of:
- obtaining material from the specific plant,
- providing an assayable form of at least a part of said plant material, detecting by thin layer chromatography a pattern of a biomarker composition of said assayable form of at least two biomarkers, said biomarkers being phytochemical compounds selected from the group of amino acids, sugars, glycosides and N-containing compounds, which after the plant has been exposed to said herbicide, either increase or decrease in concentration, are eliminated, or are newly produced, and
- correlating said pattern of a biomarker composition to a standard biomarker pattern obtained by thin layer chromatography for said specific herbicide and said specific plant,
- assessing whether said plant has been exposed to said specific herbicide. In a further aspect, the invention describes
   the above method, wherein said standard biomarker pattern is produced by:
- subjecting said specific plant to said specific herbicide,
- obtaining material from said specific plant,
- determining the phytochemical responses of said material from said specific plant for said specific herbicide, and
- hereby obtaining at least one standard biomarker pattern relating to said specific herbicide.

Another aspect of the invention is the use of the method of testing as defined by the invention. Particularly the invention concerns the testing of material from plants.

### Figures

The term "Metsulfuron (METS)" on the figures below means Metsulfuron methyl.
Figure 1: depicts the transparent shapes, i.e. the biomarker pattern of *Anagallis arvensis* L. (Primuláceae) after exposure to the herbicides Pendimethalin, Bromoxynil, Metsulfuron methyl, and Glyphosate using the TLC-system 4d (amino acids).
Figure 2: depicts the transparent shapes, i.e. the biomarker pattern of *Anagallis arvensis* L. (Primuláceae) after exposure to the herbicides Pendimethalin, Bromoxynil, Metsulfuron methyl, and Glyphosate using the TLC-system 1 (non-specific compounds).
Figure 3: shows the transparent shapes, i.e. the biomarker pattern of *Anagallis arvensis* L. (Primuláceae) after exposure to the herbicides Pendimethalin, Metsulfuron methyl, and Glyphosate using the TLC-system 2 (phenolic compounds).
Figure 4: depicts the transparent shapes, i.e. the biomarker pattern of *Anagallis arvensis* L. (Primuláceae) after exposure to the herbicides Bromoxynil, Metsulfuron methyl, and Glyphosate using the TLC-system 6 (lipids and terpens).
Figure 5: depicts the transparent shapes, i.e. the biomarker pattern of *Anagallis arvensis* L. (Primuláceae) after exposure to the herbicides Pendimethalin, Bromoxynil, Metsulfuron methyl, and Glyphosate using the TLC-system 7 (non-specific compounds).
Figure 6: depicts the transparent shapes, i.e. the biomarker pattern of *Centauréa cýanus* L. (Asteráceae) after exposure to the herbicides Pendimethalin, Bromoxynil, Metsulfuron methyl, and Glyphosate using the TLC-system 4d (amino acids).
Figure 7: depicts the transparent shapes, i.e. the biomarker pattern of *Centauréa cýanus* L. (Asteráceae) after exposure to the herbicide Glyphosate using the TLC-system 1 (non-specific compounds).
Figure 8: depicts the transparent shapes, i.e. the biomarker pattern of *Centauréa cýanus* L. (Asteráceae) after exposure to the herbicides Pendimethalin, Bromoxynil, Metsulfuron methyl, and Glyphosate using the TLC-system 2 (phenolic compounds).
Figure 9: depicts the transparent shapes, i.e. the biomarker pattern of *Centauréa cýanus* L. (Asteráceae) after exposure to the herbicides Metsulfuron methyl and Glyphosate using the TLC-system 6 (Lipids and Terpens).
Figure 10: depicts the transparent shapes, i.e. the biomarker pattern of *Centauréa* cýanus L. (Asteráceae) after exposure to the herbicides Bromoxynil, Metsulfuron methyl, and Glyphosate using the TLC-system 7 (non-specific compounds).
Figure 11: depicts the transparent shapes, i.e. the biomarker pattern of *Lólium perénne* L. (Poáceae) after exposure to the herbicides Pendimethalin, Bromoxynil, Metsulfuron methyl, and Glyphosate using the TLC-system 4d (amino acids).
Figure 12: depicts the transparent shapes, i.e. the biomarker pattern of *Lólium perénne* L. (Poáceae) after exposure to the herbicides Bromoxynil, Metsulfuron methyl, and Glyphosate using the TLC-system 1 (non-specific compounds).
Figure 13: depicts the transparent shapes, i.e. the biomarker pattern of *Lólium perénne* L. (Poáceae) after exposure to the herbicides Metsulfuron methyl and Glyphosate using the TLC-system 2 (phenolic compounds).
Figure 14: depicts the transparent shapes, i.e. the biomarker pattern of *Lólium perénne* L. (Poáceae) after exposure to the herbicides Bromoxynil, Metsulfuron methyl, and Glyphosate using the TLC-system 6 (lipids and terpens).
Figure 15: depicts the transparent shapes, i.e. the biomarker pattern of *Lólium perénne* L. (Poáceae) after exposure to the herbicides Pendimethalin, Metsulfuron methyl, and Glyphosate using the TLC-system 7 (non-specific compounds).
Figure 16: depicts the transparent shapes, i.e. the biomarker pattern of *Plantago lanceoláta* L. (Plantagináceae) after exposure to the herbicides Pendimethalin, Bromoxynil, Metsulfuron methyl, and Glyphosate using the TLC-system 4d (amino acids).
Figure 17: depicts the transparent shapes, i.e. the biomarker pattern of *Plantágo lanceoláta* L. (Plantagináceae) after exposure to the herbicides Metsulfuron methyl and Glyphosate using the TLC-system 1 (non-specific compounds).
Figure 18: depicts the transparent shapes, i.e. the biomarker pattern of *Plantágo lanceo*/*áta* L. (Plantagináceae) after exposure to the herbicides Pendimethalin, Metsulfuron methyl, and Glyphosate using the TLC-system 2 (phenolic compounds).
Figure 19: depicts the transparent shapes, i.e. the biomarker pattern of *Plantágo lanceoláta* L. (Plantagináceae) after exposure to the herbicides Pendimethalin, Metsulfuron methyl, and Glyphosate using the TLC-system 6 (lipids and terpens).
Figure 20: depicts the transparent shapes, i.e. the biomarker pattern of *Plantago lanceoláta* L. (Plantagináceae) after exposure to the herbicides Metsulfuron methyl and Glyphosate using the TLC-system 7 (non-specific compounds).
Figure 21: depicts the transparent shapes, i.e. the most frequent biomarker pattern of all tested plants after exposure to the herbicides Pendimethalin, Bromoxynil, Metsulfuron methyl, and Glyphosate using the TLC-system 4d (amino acids).
Figure 22: depicts the transparent shapes, i.e. the most frequent biomarker pattern of all tested plants after exposure to the herbicides Metsulfuron methyl and Glyphosate using the TLC-system 1 (non-specific compounds).
Figure 23: depicts the transparent shapes, i.e. the most frequent biomarker pattern of all tested plants after exposure to the herbicides Pendimethalin, Metsulfuron methyl, and Glyphosate using the TLC-system 2 (phenolic compounds).
Figure 24: depicts the transparent shapes, i.e. the most frequent biomarker pattern of all tested plants after exposure to the herbicides Metsulfuron methyl, and Glyphosate using the TLC-system 6 (lipids and terpens).
Figure 25: depicts the transparent shapes, i.e. the most frequent biomarker pattern of all tested plants after exposure to the herbicides Metsulfuron methyl, and Glyphosate using the TLC-system 7 (non-specific compounds).

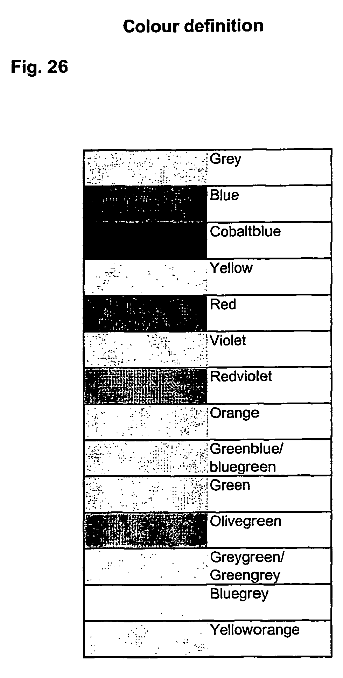

Figure 26: shows the colour definitions used for the transparent shapes.

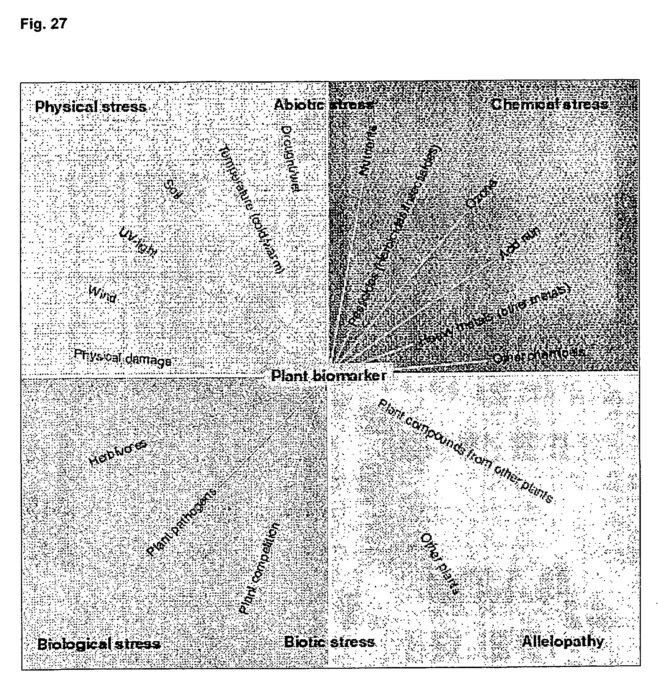

Figure 27: is an overview of different types of stress.

### Detailed description

By the present invention it has become possible to detect whether a plant has been exposed to a herbicide by applying a simple and highly sensitive method of testing.

The application relates to a method for testing whether material from a living organism has been exposed to stress, comprising the steps of:
- obtaining material from a living organism,
- providing an assayable form of at least a part of said living organism material,
- detecting a biomarker pattern of said assayable form of at least two biomarkers, said biomarkers being compounds which after the living organism has been exposed to stress, either increase or decrease in concentration, are eliminated, or are newly produced, and
- correlating said biomarker pattern to standard biomarker pattern, and/or
- correlating said biomarker pattern to a standard pattern,
- assessing potential stress exposure for said living organism material.

In principle the application relates to any living organisms for which it is desired to test whether said organism has been exposed to stress, i.e. external stress. To simplify the description the following discussion concerns material for which the living organism is a plant. Further, the invention particularly discloses the use of biomarker patterns in the control of herbicide spraying.

The present application is based on the recognition that the phytochemical compounds in plants exposed to stress, such as pesticides, are related to and depending on the pesticides used and their modes of action in the plant. The present inventor has found a reproducible pattern of the composition of the phytochemical compounds in plants after exposure to a stress factor, such as a pesticide, said pattern being unique to the specific stress factor, and unique to the individual plant family, more preferred the individual plant species, such as to individual plant varieties. The unique pattern is a so-called fingerprint of the effect of a specific pesticide in the plant in question, i.e. the specific plant to be tested. Thus, the present invention offers an opportunity to assess/determine whether a plant has been exposed to stress factors, such as pesticides in spite of the fact that the potential exposure cannot be assessed by visual inspection of said plant as visual signs. By the term "visual inspection" is meant an ordinary visual inspection with the naked eye, whereby morphologigal changes, such as changes in colour, wiltering etc. of the plant may be inspected.

Certain new chemical compounds may be produced in the plant after exposure to stress, or the concentration of already existing compounds may change, for example by an accumulation of certain chemical compounds in the plants. Furthermore, the pattern may also be related to a decrease or even an elimination of chemical compounds in the plants after exposure to stress. These changes of concentration of compounds, elimination of compounds and/or production of new compounds after stress exposure may be due to changes in the biochemical pathways of plants.

Accordingly, a biomarker pattern is the pattern of the composition of phytochemical compounds, i.e. endogenously produced compounds, in the plant after exposure to a stress factor, i.e. an external exposure, and said pattern is unique for each type of stress factors, such as pesticides, or for a group of stress factors.

In one aspect of the invention, the compounds present in the plants after exposure are the same as before exposure, but the concentration of the individual compounds is different, whereby a new pattern of the phytochemical compounds has arised after exposure.

### Time/age/sensitivity

In another aspect of the invention the presence of phytochemical changes and the extend of sensitivity of the plant to stress exposure is dependent on the age of the plant. Young plants tend to be more sensitive to exposure of stress, such as herbicides, than older plants. This means that a biomarker pattern can be detected at an earlier stage after the time of exposure in a young plant as opposed to the later stage of detection of a biomarker pattern in an older plant. This knowledge of the correlation between plant age and the time neccessary for the plant to develop a biomarker pattern (i.e. sensitivity) may be used to determine how long ago a certain plant were exposed to stress factor(s). Due to their high sensitivity young plants show lower stability of the biochemical changes, i.e. the biomarker pattern is more stable in older plants and may be observed throughout the remains of the life of the older plant. However, younger plants have a higher sensitivity to stress and also a higher mortality rate. Fewer species of young plants will survive stress exposure the first weeks after emergence, while older plants are less affected.

Accordingly, the present invention takes advantage of a number of parameters, such as the phytochemical responses and the time after stress exposure with which they occur, the physiological effects, the types, numbers and concentrations of compounds biosynthesised in plants after exposure to pesticides.

In one aspect of the invention the pattern of the composition may relate to at least 2 biomarkers. In another embodiment of the invention the pattern of the composition relates to at least 3 biomarkers, such as at least 4 biomarkers, for example at least 5 biomarkers, such as at least 6 biomarkers, for example at least 7 biomarkers, such as at least 8 biomarkers, for example at least 9 biomarkers, such as at least 10 biomarkers.

By the term "standard biomarker pattern" is meant a pattern of the composition of compounds present in a plant after exposure to known stress factors. According to the invention the biomarker pattern of the unknown compounds, i.e. the above described biomarker pattern, is correlated to a standard biomarker pattern, also referred to as a "most frequent pattern". In order to interpret the biomarker patterns of test material that has been exposed to unknown stress factors, it is a prerequisite to provide standard biomarker patterns, i.e. most frequent patterns. By the latter is meant the same type of biomarker pattern obtained after exposure to a stress factor in more than 50% of the cases of exposure, such as 60%, for example 70%, such as 80%, for example 90%. The biomarker patterns of test material that has been exposed to unknown stress factors may then be correlated to standard biomarker patterns. The standard biomarker pattern may be obtained for one particular stress factor or for a combination of at least two different stress factors.

In another embodiment of the invention it is possible to correlate the biomarker pattern not only to the standard biomarker pattern as described above, but also to a standard pattern. By the term "standard pattern" is meant a pattern of the composition of compounds present in a plant before exposure to any stress factors. Such plant may also be referred to as "stress free" or "a 0 plant". The compounds present in a "0 plant" may be described as naturally occuring compounds, i.e. compounds naturally present in the plant before any stress exposure.

It is possible to investigate the standard pattern for material from a living organism that has been exposed to stress comprising the steps of:
- subjecting a living organism to known types of stress,
- obtaining material from said living organism,
- determining the chemical responses of said material from said living organism for each stress type, and
- obtaining at least one standard biomarker pattern relating to said stress types.

The description below applies to both a method of providing a standard biomarker pattern as well as to a method of testing whether material from a living organism has been exposed to stress.

The material on which the testing is performed may be from any living material, such as from animals, for example mammals, soil invertebrates and insects, or from thallophytes, such as fungi or algae. However, in a preferred embodiment of the application the material from a living organism is plant material.

In a preferred embodiment the material is selected from plants, fungi or algae. The following is a description of one embodiment, wherein the material from a living organism originates from plants. The description of this embodiment using plants, also relates to other embodiments, wherein the material from a living organism is not plant material.

Thus in one aspect the method of testing is to determine the composition of the chemical biomarker pattern after exposure to stress.

The plant material of the invention may be selected among any plant or plant cells. In one embodiment of the invention the plant material is chosen from, but not limited to dicotyledons or monocotyledons. According to the invention the dicotyledonous plants may be selected from the families of Asteráceae, Brassicaceae, Lamiaceae, Polygonaceae, Papaveraceae, Primuláceae, Plantagináceae and Scrophulariaceae and the monocotyledonous plants may be selected from the families of Poáceae.

According to the invention the plant material used to perform the method of testing may be the entire plant or it may be at least a selected area of any part of the plant. The selected area of the plant may be an area such as from at least flowers, shoots, leaves, stems, roots, seeds, pollen, rhizomes, stamens, sepals, petals, carpels, styles, stigmas, microsporangia, anther, fruits, cotyledons, hypocotyle, epicotyle, xylem or/and phloem (wood), periderm (bark), buds, flower buds, cones, cone scales, tubers, bulbs, root nodules, resin or sap.

Once a sample of the plant material is obtained, a second step in the method according to the invention begins. It is an object of the present invention to provide a method of testing, wherein the plant material used is in a form suitable for assaying. One such suitable form may be a liquid form, for example a liquid suspension. A liquid suspension of the plant material may be obtained by applying extraction solvents, such as ethanol to the plant material. The extraction solvents ensure that all compounds form all chemical groups present in the plant material are extracted. The assayable plant material may be fresh or non-fresh.

In a preferred embodiment of the invention the plant material is fresh. The fresh material may be used for analysis immediately after harvesting said material or it may be used for analysis up to a few minutes after harvesting. It is preferred that the fresh material is used as soon as possible after harvesting to avoid break down processes, such as enzymatic breakdown.

In one embodiment the plant material is frozen. The frozen plant material may be frozen up to the point of analysing, such as frozen for a period of at least 2 years and it may be defrosted prior to performing the test. However, it is preferred that the frozen plant material is used for analysis immediately after being removed from the cold storage.

In another embodiment of the invention the plant material is dry. The drying process may be accounted for by air, or nitrogen, or it may be a freeze drying process, such as nitrogen dried. Additionally the plant material may be heat dried, such as sun dried. It is important that the plant material is substantially dry, and the length of the drying process is dependent on the type of plant material.

In one aspect a method of testing whether plants have been exposed to pesticides is presented. However, the present application further relates to a method of testing having an improved sensitivity, i.e. detection limit. By detection limit is meant the lowest possible concentration of for example pesticides the test of the invention is capable of determining. Thus, not only is it possible to detect the exposure to pesticides, but it is possible to detect the exposure, of for example pesticide concentrations below the recommended level of dosage. The "recommended dose" is the effective dose needed to obtain a result. According to the invention the method of testing may be performed on plants being exposed to less than 10% of the recommended dose, preferably down to less than 5% of the recommended dose, more preferably down to less than 1% of the recommended dose without any visual effects of the exposure on the plants tested.

The length of the time period before the plants react to the stress exposure may be dependent on numerous factors, such as the species and age of the plant. Some plants may recover from an exposure, and the detection may take place before such a recovery. However, it may be possible to detect biomarkers after the plant has recovered from the exposure. Without being bound by theory the detection of biomarkers may for some plants be possible throughout the entire life span of the plants, whereas the detection of biomarkers of other plants may only be possible within a certain time frame. This of course may depend on the nature of the plant species and of the stress factors as such, for example the concentration level of pesticides.

Accordingly, the detection of biomarkers may be possible as long as the plant is living. This may be between less than 1 day and up to 35 days after exposure, such as between 1-30 days after exposure, for example between 5-25 days after exposure, such as between 10-20 days after exposure, for example between 12-18 days after exposure.

The detection of biomarkers in a plant may in one aspect of the invention serve the purpose of an "early warning" signal of stress exposure before any visual signs thereof appear on the plant.

It has been reported that when plants are exposed to stress they may react by changing their phytochemical composition. The present invention presents a method by which reproducible biomarker patterns are obtained, thus providing analytical tools for the establishment of exposure to and identification of known as well as unknown compounds. There is a variety of stress factors that may all have an impact on the chemical composition of plants. The plant may be exposed to more than one stress factor, wherein in one embodiment the effect of the exposure is synergistic and thus results in a biomarker pattern reflecting the synergistic effect of the individual stress factors. In another embodiment, wherein the plant may be exposed to more than one stress factor, the resulting biomarker pattern reflects the antagonistic effect of the individual stress factors. It is within the scope of the invention to develop a standard biomarker pattern for any combination of stress factors.

According to the application one of the stress factors is abiotic, such as chemical stress and/or physical stress.

In the present context chemical stress may be caused by pesticides, such as herbicides. Herbicides are all designed to kill plants by altering and effecting the biochemical homeostasis of the plant cells. Plants react to the exposure of herbicides by producing or decomposing phytochemical compounds. They may also react by changing the concentration of already existing compound(s). The resulting effect on the plants is dependent on the individual mode of action of the herbicide.

In one aspect of the application the method of testing for the exposure of pesticides relates to herbicides selected from a group consisting of Glyphosate, Bromoxynil, Pendimethalin and Metsulfuron methyl, all representing different modes of action on the target plants. These herbicides are all widely used in Northern America and Western Europe for the control of broad-leaved plants and grasses.

Glyphosate (GLY) is a non-selective herbicide that controls emergent annual and perennial broad-leaved plants and grasses (Tomlin, 2000; Trottier *et al.*, 1990). Glyphosate inhibits the activity of the EPSP-enzyme (5-enolpyruvylshikimate-3-phosphate) of the aromatic acid biosynthetic pathway in plants. It is absorbed through the wax cuticle on the leaves and a rapid translocation occurs via phloem to roots, rhizomes and apical meristems. It is degraded by rapid microbial action, with a half-life of 3-5 weeks. It is non-volatile and does not degrade photochemically. The water solubility is 11.6 g/l at 25 °C. It binds strongly to soil particles and hereby it is immobile unless transported with the soil.

Bromoxynil (BRY) is a selective herbicide with some systematic activity (Thomson, 1989; Tomlin, 2000). The herbicide is absorbed by the foliage through cuticular penetration. Bromoxynil kills by inhibition of photosynthesis and plant respiration in annual broad-leaved plants. It degrades rapidly in most soil types, with a half-life in the order of two weeks which can be considerable reduced at low temperatures. It is water-soluble (130 mg/l), potentially harmful to fish and aquatic invertebrates for which it is toxic if it reaches water bodies (Muir *et al*., 1991).

Pendimethalin (PEN) is a selective herbicide that inhibits cell growth by inhibiting cell division of any and all plant cells by acting as a mitotic toxin. It is absorbed by roots and leaves, but initially limits root growth, such as the development of lateral or secondary roots (Tomlin, 2000). Pendimethalin is moderately persistent in moist sandy loam (half-life 50 days) to highly persistent in moist silty soil (half-life 140 days) and in dry silty clay loam (250 days). It is a very stable herbicide except when it volatilises from moist soil surfaces (Barrett & Lavy 1983). The water solubility is 0.3 mg/l at 20 °C. Thus, it is likely to be transferred to other environmental compartments although it may move with soil particles to water bodies where it is toxic to fish (Thomson, 1989; Tomlin, 2000).

Metsulfuron methyl (METS) is a potent inhibitor of plant growth used on wheat and barley crops for the control of broad-leaf species and the suppression of few grasses. The herbicide is taken up by the foliage or the roots and translocated via xylem and phloem. Metsulfuron methyl is a selective herbicide that acts by inhibiting the enzyme acetolactate synthase (ALS) which catalyses the synthesis of the three branched-chain amino acids valine, leucine and isoleucine (Moberg & Cross 1990). The precise mechanism of action is unknown, but soon after herbicide application, plant cell division quickly stops, and death occurs within one to three weeks. The accumulation of ALS substrates (e.g. α-ketobutyrate) in leaves may be responsible for the cessation of the plant growth with decreased production of new leaves and reproductive organs (Bestman *et al.,* 1990). Metsulfuron methyl is mobile in most soil and the mobility is enhanced as pH increases (Beyer *et al.*, 1988; Blair 1988).

All the above mentioned herbicides are currently applied to major crops, such as maize, wheat, barley, soybeans, oats, peas, potatoes and tomatoes. When applying herbicides to a cultivated field of crops adjacent non-target areas may be affected by herbicides as well.

Other pesticides than the ones mentioned above are also within the scope of the invention. They are described in The Pesticide Manual (twelfth edition) version 2.0, British Crop Protection Council (2000-2001). For example insecticides, acaricides, nematicides/vermicides, rodenticides and fungicides.

Further, in the present application the method of testing is applied to plants potentially being exposed to physical stress, such as temperature, wind, UV light, physical damage, soil quality and soil moistness.

In another aspect of the application the stress factors may be biotic, such as biological stress and/or allelopathy. The term "biological stress" is meant as stress and possibly visual damage caused by herbivores, plant pathogens and/or competition from other plants. The latter may also be referred to as allelopathy, such as competition from other plants and/or chemical compounds of other plants effecting/stressing the plant on which a test is performed.

There is a difference in the sensitivity of plants against various stress factors, and it is therefore in one embodiment recommended to use sensitive plants. This allows for the detection of pesticides which have been applied to target plants in even very small concentrations. An example of a model-plant is *Anagallis arvensis*. This particular model-plant is discussed below in the experimentals section.

The composition of the biomarker pattern may be phytochemical. The term "phytochemical" relates to any chemical or compound or nutrient or fundamental compound present in plants. There are a vast number of compounds present in plants. Some of the compounds are readily detectable under circumstances where said plants are not exposed to stress. If, however, plants are exposed to stress the biochemical pathways within the plant cells may be effected. The influence of stress on biochemical pathways may lead to an increase of or change, such as an elimination in the concentration of already existing compounds, or it may lead to the production of compounds not normally present in plants not exposed to stress (see above). In one embodiment the phytochemical is a substance, or at least part of a substance, or a derivative of amino acids, amines, sugars, flavonoids, phenolic compounds, sapogenins, saponins, iridoids, glycosides, alcaloids, alkaline alcaloids, N-containing compounds, S-containing compounds, P-containing compounds, O-containing compounds, such as any fundamental element, terpenoids, lipids, steroids, cartenoids, quinones, coumariners, and nutrients, such as any compound necessary for the plant to survive, for example salts.

By the term fundamental compound is meant any compound depicted in the periodical system.

In the method of testing according to the application the assessment of potential stress exposure for material from a living organism, such as plant material may be qualitative and/or quantitative and/or semi-quantitative. In one embodiment the assessment is qualitative, and the biomarker pattern is analysed based on the composition of compounds present, i.e. the presence or absence of a particular compound. In a further embodiment the assessment is quantitative and the pattern of the composition of compounds analysed is related to the actual concentration of compounds present since the intensity of the spots (concentration of the compounds) reflects the concentration of a pesticide, for example. The quantitative evaluation may be performed by using a scanning-densitometer. In yet another embodiment the assessment is semi-quantitative. By this is meant an assessment which is partly quantitative by the means of either visual inspection or use of an apparatus. The concentration of the sample may be determined as an approximate intensity value within a given interval or point system.

The chemical analysis of pesticides is very difficult when the presence of the pesticide in the environment is low. Furthermore, it is very expensive to perform chemical screenings for chemical compounds, such as pesticides and/or their decomposition compounds and/or adjuvants present in pesticides. By the present application it is now possible to determine different stress factors, such as pesticides by a simple and affordable method of testing.

In one embodiment of the application the method of testing, comprises the following steps:
- contacting an assayable form of said plant material with a support for receiving said plant material,
- subjecting said support to a solvent,
- optionally drying said support,
- optionally contacting said support with a chemical reagent,
- obtaining a biomarker pattern of said assayable form.

In the present context an assayable form may be a liquid, or a liquid mixed with solids, such as liquids mixed with salts.

The support for receiving the material may be a solid material or a less solid material, such as a soft material, for example a liquid material. The support may be pretreated with a substance capable of promoting reactions when put into contact with the plant material. Said reactions may be for example be radioactive, fluorescent, or immunological.

In one aspect the biomarker pattern is detected by the use of commercially available techniques known to the skilled artisan, such as High Performance Liquid Chromatography (HPLC) or gas chromatography or mass spectrometry (MS), or a combination of analytical methods. For example a densitometric evaluation of thin-layer chromatograms using a densitometric scanner/videoscan may be employed. It is important that the same method of analysing is applied when detecting both the standard biomarker pattern, and the biomarker pattern resulting from the exposure of unknown stress factors.

In a preferred aspect the biomarker pattern is detected by the use of Thin Layer Chromatography (TLC) (Stahl, 1956). TLC is a well-known simple chromatographical separation method. The technical advantage of using TLC techniques compared to e.g. HPLC, is the visual colour reaction of the plant biomarker. Additionally, TLC is a cheaper analysis compared with other analytical analysis. In one embodiment the TLC method is circular.

Other relevant biomarker detection methods are infrared spectrophometry, spectrophometry, refractrometri, nuclear magnetic resonance, and electrophoresis. Radioactive compounds may be used in said methods. For example a "film" of radioactivity may be placed over a TLC-plate, whereafter a pattern of biomarkers emerge where the radioactive compounds are placed.

When using the method of Thin Layer Chromatography testing according to the invention, it comprises the following steps:
- contacting an assayable form of said plant material with a TLC-plate,
- subjecting said TLC-plate to a solvent,
- optionally drying said TLC-plate,
- optionally contacting said TLC-plate with a chemical reagent,
- obtaining a biomarker pattern of said assayable form.

In a further aspect the biomarkers are separated by the means of antibodies possible attached to support or antibodies in the solvent.

In order to verify the shapes of the finger print of the composition i.e. the change of the composition of the biomarkers or phytochemical changes, different TLC-plates, solvents and chemical reagents are used. This results in different colour reactions, which may be visually inspected. The TLC-plates used in the test of the invention are all commercially available TLC-plates, and may be made from cellulose or silica gel. The types of TLC-plates chosen for the test are selected according to the plant species tested, and to the specific compounds of which it is desired to determine their existence or absence in the plant material.

In the present context the term "solvent" is meant to cover one substance or a combination of two or more substances, wherein the solvent may be a combination of liquid and solid and gas substances. A solvent may be a reagent, an eluent or an extractionmedia. The latter three may be in a solid or liquid physical state, or they may be in the form of a gas.

In one embodiment of the invention an extract of material from a plant is provided. The extraction may be performed under cold or warm temperatures, such as by the means of ultrasound, or stiming and according to the invention the extraction solvent may be an petroleum-ether extraction solvent, or a 10 % acidic acid in 96 % ethanol extraction solvent. In another aspect the extraction solvent is 75 % ethanol as described in the experimental section below. The extraction may be performed on fresh or non-fresh plant material.

According to the invention the solvents and the support may have different polarities, such as between -0.1-10, for example between 2-8, such as between 4-6 as defined by Snyder, (1974). The polarity of the solvents and support is crucial for the resulting biomarker pattern.

Once the extract of the plant material has been placed on a TLC-plate the TLC-plate is placed in a TLC chamber containing a chemical eluent. The eluent is absorbed by the plate material and this initiates the development of the test. The biomarker compounds react with the plate material and the eluent. All biomarkers have different affinity to the plate material and to the eluent. Thus, the biomarkers will appear in different positions on the plate material. The higher affinity the biomarkers have to the eluent the further they will migrate on the TLC-plate. The first step of "developing" the plate, i.e. the reaction of the plant material with the plate material and element may be a period of 120 minutes, such as 90 minutes, for example 60 minutes. In one embodiment of the invention the solvent comprises the upper-phase of n-butanol and formic acid in a ratio of 2:1. The solvent may only be stable for up to 1 day and therefore has to be renewed daily.

In another embodiment the solvent comprises n-butanol, acetic acid and water in a ratio of (4:1:5). The solvent is stable for several days and is preferably stored in a cool place.

The TLC-plate may then be air-dried and a chemical reagent may be brought in contact with the TLC plate, such as by spraying. The type of reagent of the invention may vary according to the type of TLC-plate and the type of biomarker. As the reagent of the invention is applied to the dried TLC-plate a unique and reproducible colour reaction develops. According to the invention the colour reaction may confirm or reject the presence of specific biomarkers after exposure to stress, such as herbicides.

Accordingly, a purpose of the invention is to provide a method of testing, wherein the biomarker pattern is obtained as a result of the specific combination of parameters, such as said support, for example TLC-plates, said solvent and said chemical reagent. The specific test combinations are described in detail in the Experimentals section below.

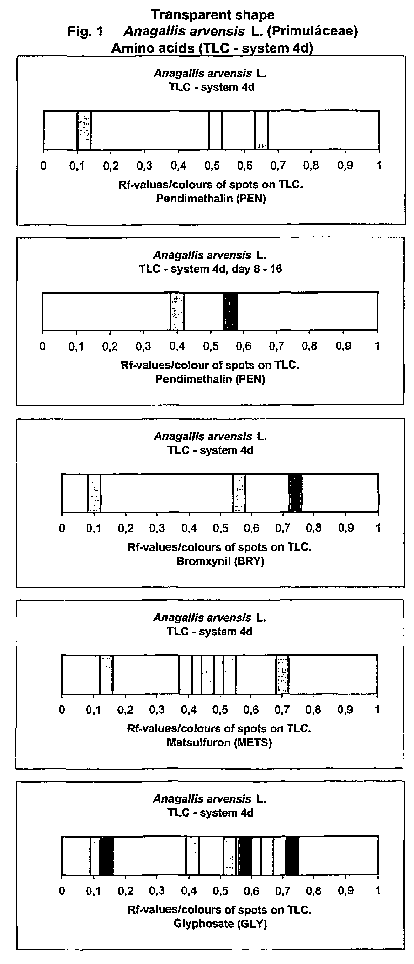

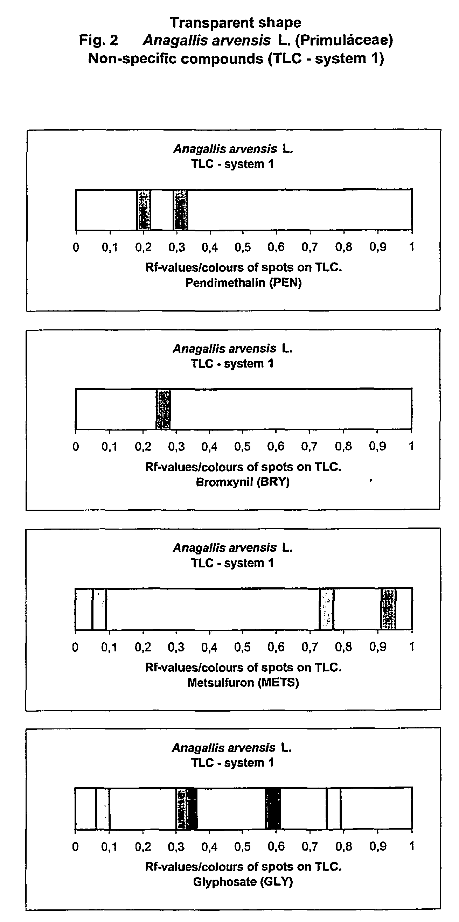

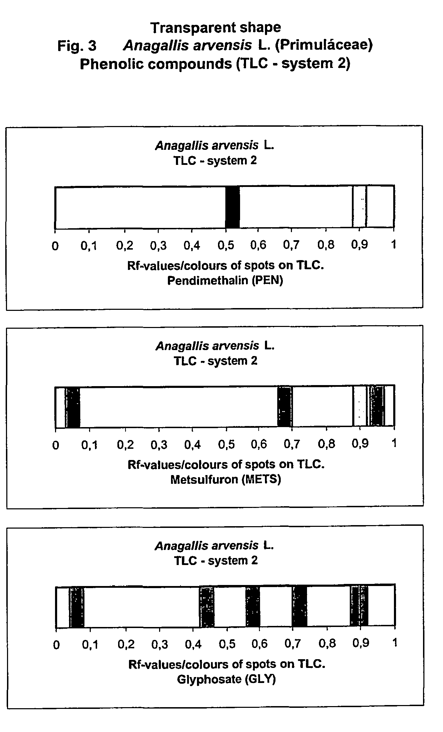

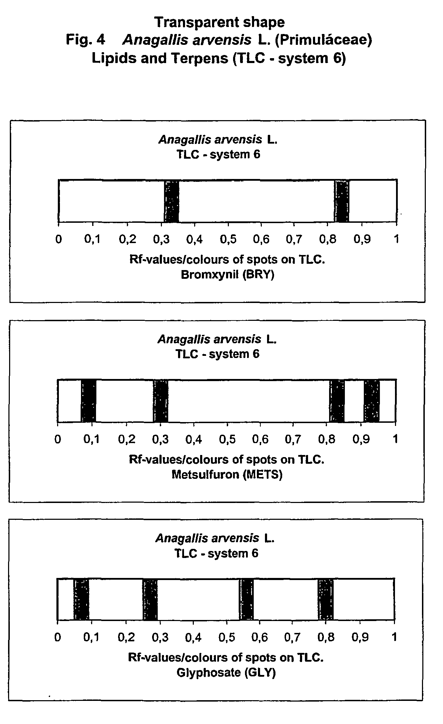

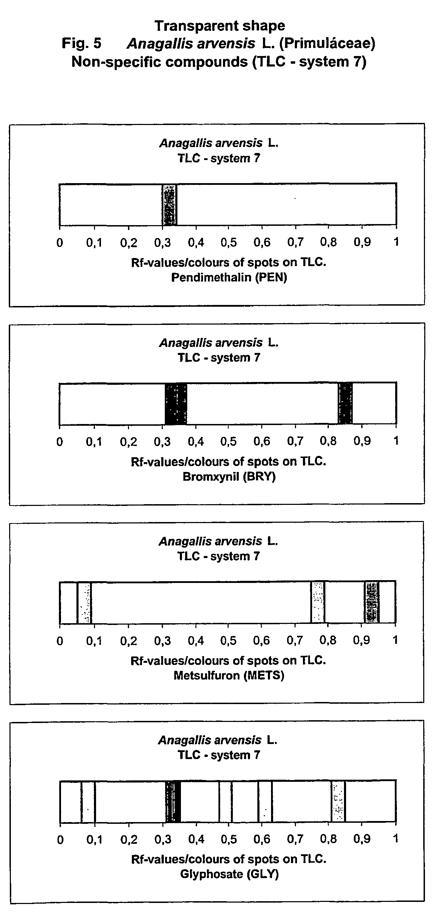

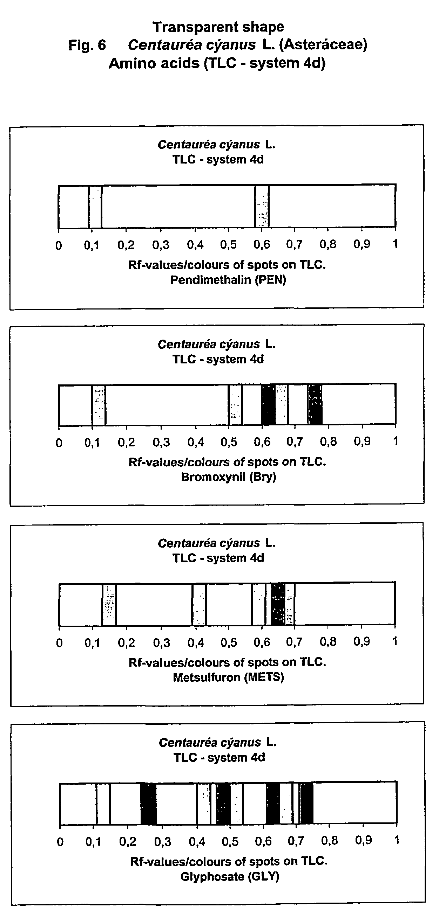

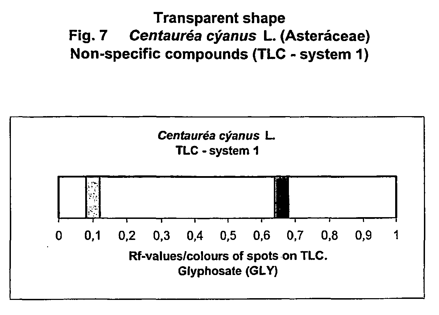

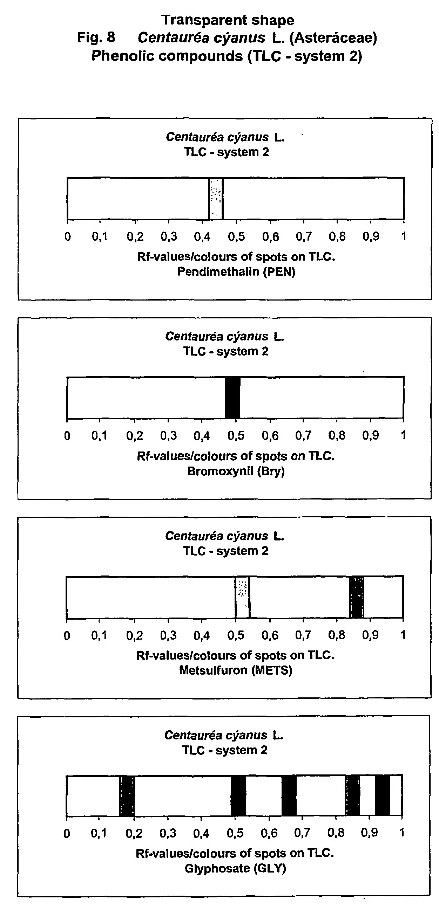

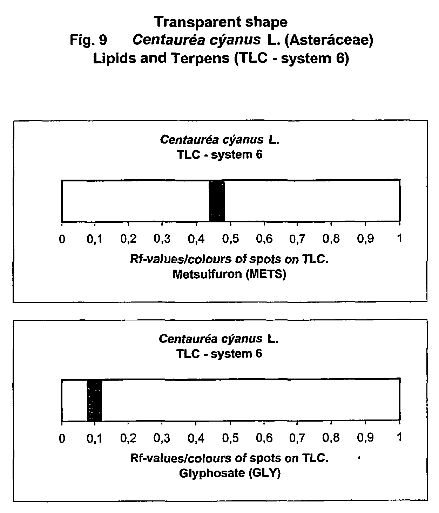

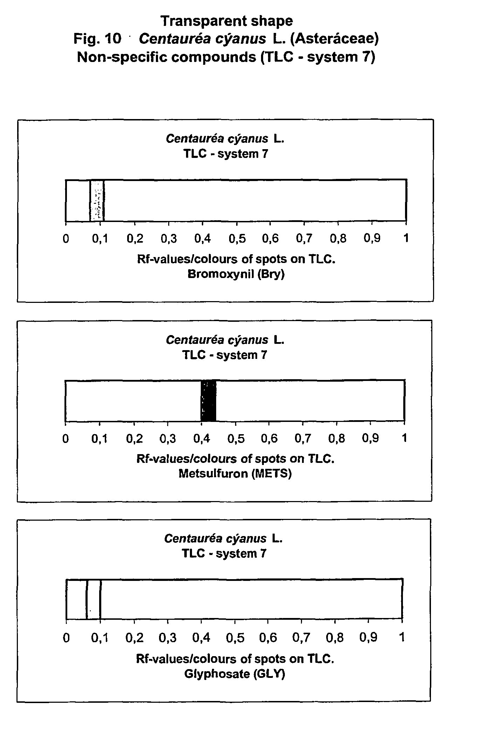

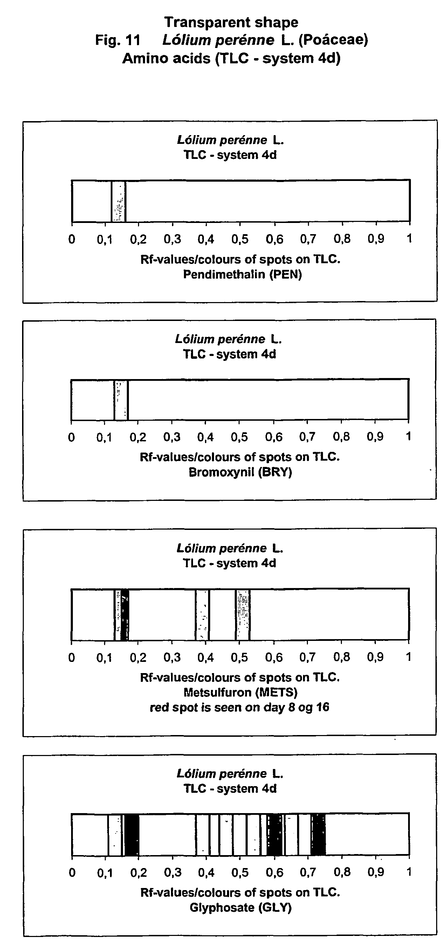

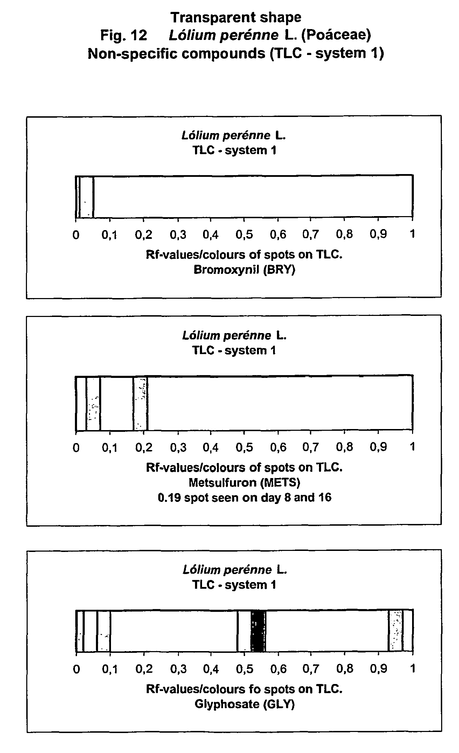

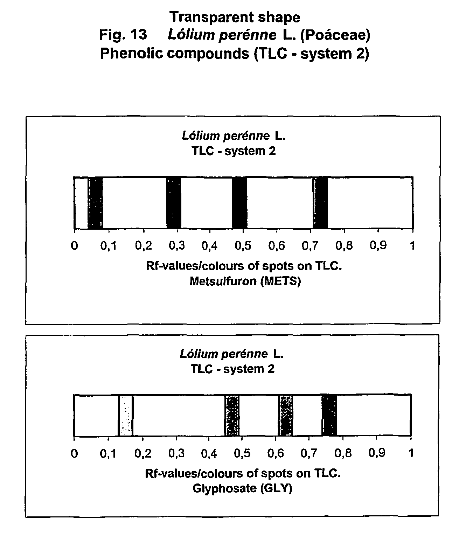

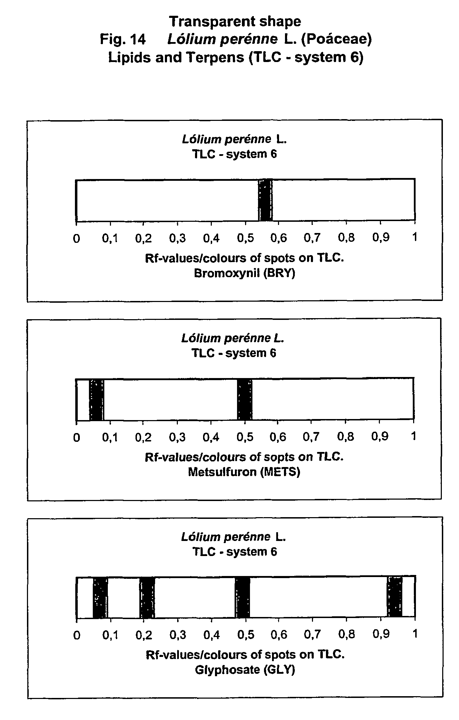

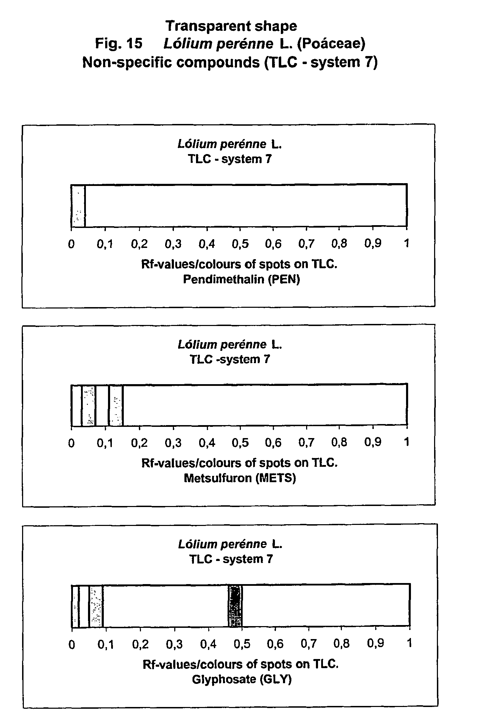

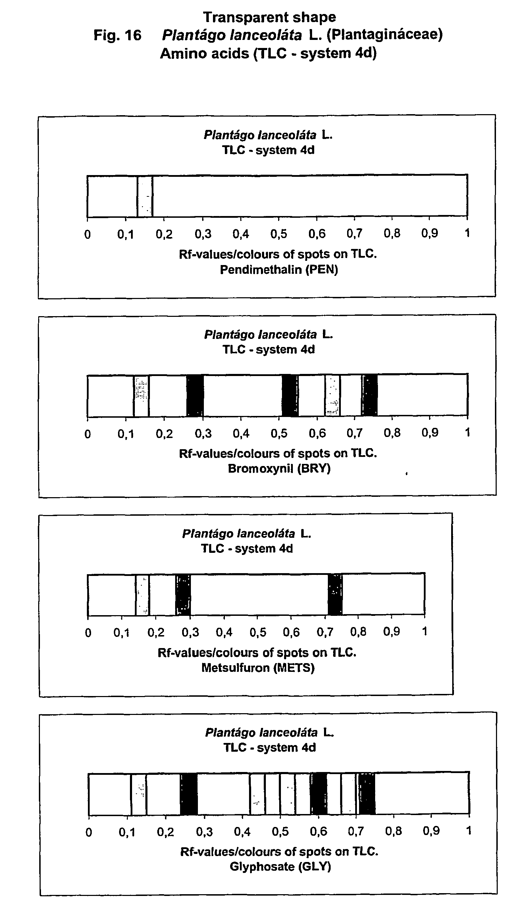

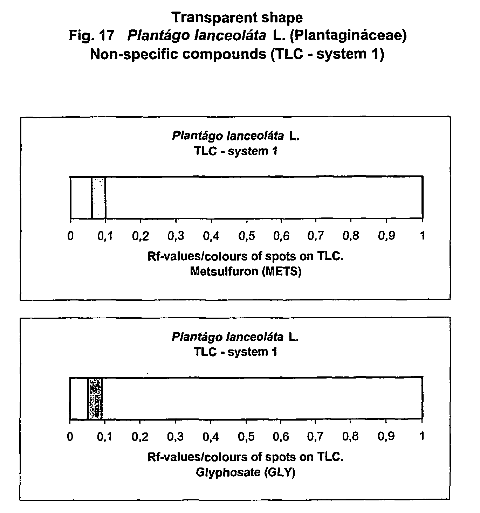

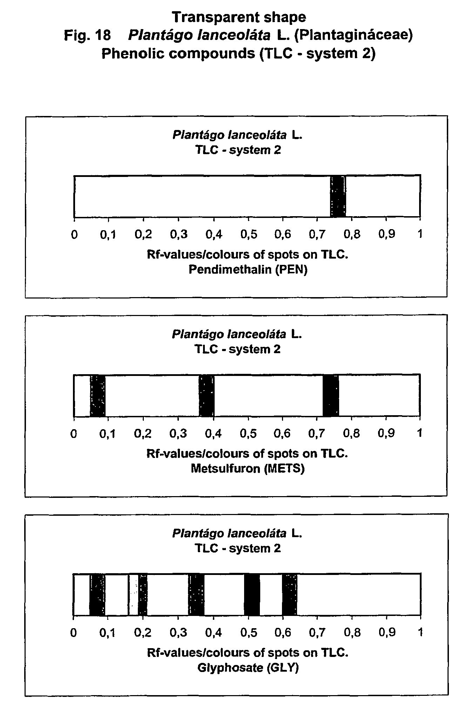

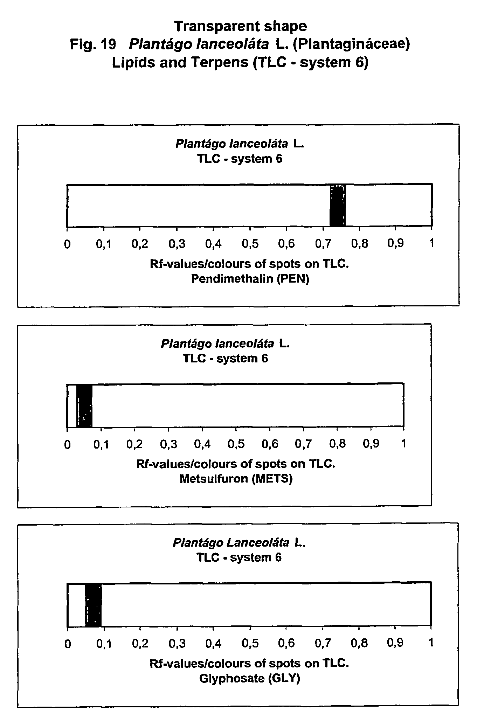

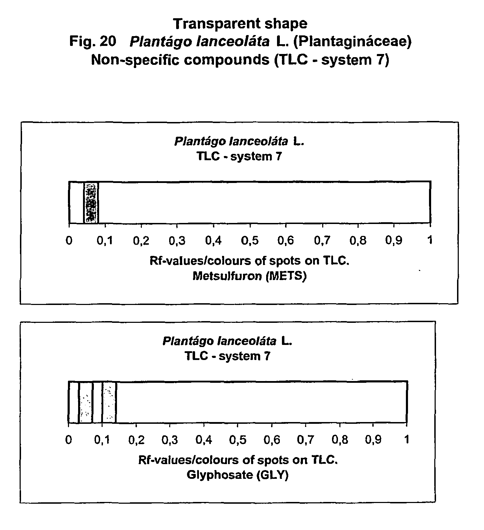

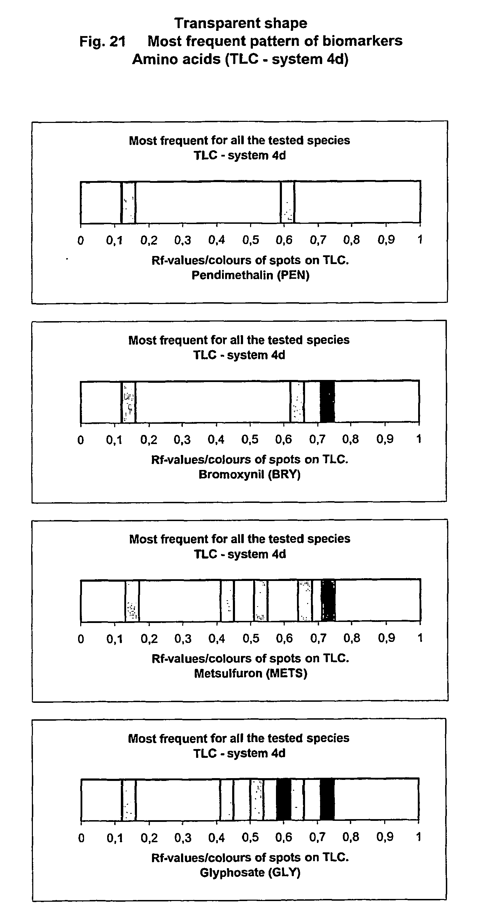

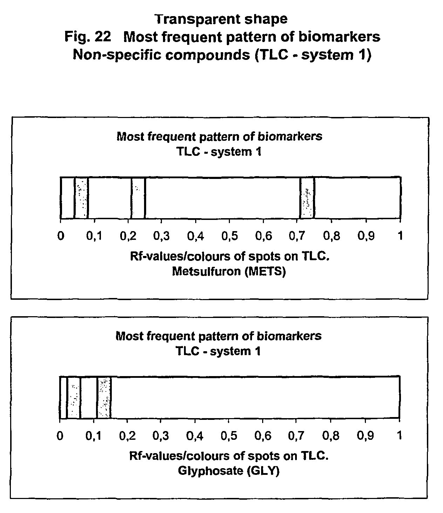

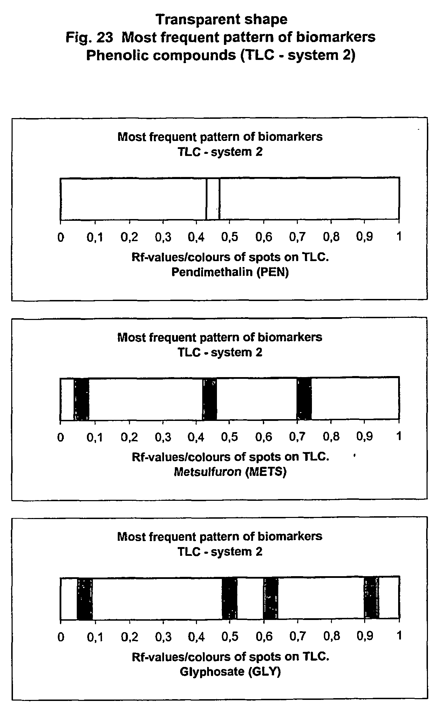

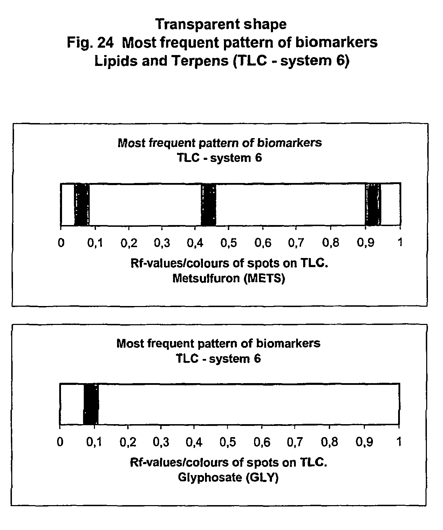

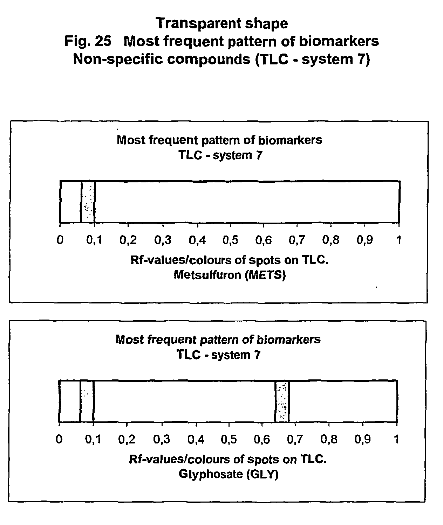

When using the TLC in the method of testing a qualitative effect of stress factors to plants Rf values are applied. Rf values are defined as the distance of the centre of the spot (i.e. compound) from the start point divided by the distance of the front of the solvent from the start point (Stahl, 1969). The Rf values are a supplement to the results obtained by the established biomarker patterns illustrated in the figures 1-25 below.

Further disclosed is an assay kit for the determination of whether material from a living organism has been exposed to stress, comprising,
- a support for receiving an assayable form of at least a part of said material from a living organism,
- optionally at least one reagent specific for the biomarkers to be detected, and
- optionally detection agents for displaying the biomarkers of said assayable form.

It is a purpose of the application to lower the costs and time of the testing procedure, and at the same time provide a method of testing having excellent sensitivity. The assay kit is for all practical purposes to be used as a field test, or as a laboratory test. One object is to have an easy accessible test to be used commercially or on a private scale. Thus, the assay kit is practical and portable in size and easy to operate. The test material is brought in contact with a support for receiving said material. The test material is in an assayable form, for example in the form of a liquid suspension.

The assay kit is used on material from plants, and it may comprise:
- Thin Layer Chromatography (TLC) plates,
- eluents,
- reagents,
- hand-press,
- micro pipettes,
- optionally an UV-lamp,
- optionally a heater,
- at least one standard biomarker pattern,
- at least one standard pattern.

The assay kit is envisioned as a practical and mobile test system. The individual steps of the test may be performed in the field, and does not require any particular technical skills of the person performing the test. The test may be completed in less than 1 hour, such as 45 minutes.

The assay kit used on plant material comprises at least one TLC-plate. The test material is brought into contact with a solid support, such as a TLC plate comprising an adsorbent material capable of separating the mixture of compounds of the test material. A hand-press may be used to press out the sap of the plant material. The assay kit also comprises containers of solvents, such as solvents described below. Containers, for example a bottle shaped container with a spraying head, holding reagents sprayed onto the TLC-plate(s) are also part of the present test kit. In another embodiment the assay kit comprises containers holding agents wherein the TLC-plates is placed. The containers may vary in size depending on the type of application in question. For repetitive tests larger containers may be preferred. In one aspect the assay kit is disposable, i.e. the individual components in the assay kit is used only once. However, in another aspect one or more of the individual components of the assay kit is recycled. Different embodiments of the assay kit are envisioned. In one embodiment all components of the assay kit are contained in one pocket size unit. In another embodiment the UV-lamp and/or the heater are separated from the assay kit as a unit. In another embodiment the UV-lamp and/or the heater are excluded from the assay kit and method as a whole. Further, micro-pipettes including holders are optionally included in the assay kit unit. The colour reactions developed on the solid supports, such as TLC-plates of the assay kit are compared to one or more colour comparators, such as the standard biomarker pattern and/or standard pattern of the invention. The colour comparators may be in the form of a folder of colour charts enclosed in the assay kit, and it is preferred that the size of the colour comparators (standard biomarker patterns and/or standard patterns) are equal to the size of the solid support, such as the TLC-plate(s).

In yet another aspect an immunological test, such as a "dipstick" is used.

Another object of the application is the use of a method for testing whether material from a living organism, including plant material, and/or a method of providing a standard biomarker pattern for material from a living organism, including plant material has been exposed to stress. The application also relates to the use of an assay kit for the determination of whether material from a living organism, such as plant material has been exposed to stress.

One of such uses may in the control of gene modified plants. It is envisioned that the biomarker pattern of plants being genetically modified may be determined by using the present test method. Plants may be genitically modified to become resistant to pesticides, and such gene modified plants may produce biomarkers that differ from biomarkers of non-gene modified plants. In one embodiment the biomarker patterns of gene-modified plants and non-gene-modified plants, which have not been exposed to pesticides, are compared.

In another aspect the application may be used in the control of the geographic distribution of pesticides. Non-target habitats adjacent to cultivated fields may be affected by pesticides during application. This exposure may occur due to over-spraying, or through spray drift from the application on target crops adjacent to wild-life habitats. It may also stem from pesticides being run-off or washed-off. Pesticides are able to travel considerable distances by air, either by drift or by volatilisation.

In a further aspect the application may be used by the farmer to assess the optimal effect of a given pesticide on plants being exposed to reduced levels of said pesticide. In this way the farmer is able to determine at what minimum dosages of pesticide a plant is still responding, and may thus be able to reduce the amount of pesticide necessary to obtain a given effect in the plant.

In yet another aspect the application may be used in food quality control, such as in the control of farmers produce, such as crops. Particularly the application may be used for the control of whether organic crop has been exposed to stress, such as herbicides. It is important to be able to determine whether organic crops are free from residues of chemicals, such as herbicides and/or defoliating agents and/or growth regulating agents, such as respiration and germination inhibition agents, growth retarding agents, root formation agents, flowers and fruit formation agents, germination promoting agents, flowering delaying agents, thinning out agents, hold-on compounds and grafting agents, or free from residues of chemicals used for: the control or treatment of plant diseases, wood destroying fungi, unwanted plant growth, growth of algae, slime promoting organisms in paper pulp, animals capable of damaging utility-and cultivated plants, vermin on domestic animals, infested cereal, cereal products, seeds and feed-stuff, textile infestant, infestant of lumber and woodwork, insects, snails, mites, rain worms, rabbits, water voles, moles, mice and rats, or free from residues of chemicals for the prevention of damages caused by vermin and chemicals for the exclusion of vermin from specific geographical areas.

Conventional control methods of testing for residues of for example herbicides include labour intensive and expensive analytical methods, based on gas chromatography or liquid chromatography, such as HPLC. However, since the active chemical groups of most herbicides, defoliating agents and/or growth regulating agents are being broken down to residual concentrations below detection limit, the task of detecting break down products requires the performance of several different chemical analysis for every individual herbicide, defoliating agents and/or growth regulating agent.

To overcome the above obstacles the present application provides a simple, cheap and reliable test method. The method may be applied in the fields, literary speaking, by commercial or official control units, or it may be used by private consumers. It is known that a change in the chemical composition of plants plays a vital role in food quality and thus in the general health. Compounds in plants the quality of for example crops. A change in the chemical composition of plants changes the quality of said plant for feeding purposes. A further object may be to contribute to document and assess the changes in the chemical composition, i.e. biomarker pattern of plants exposed to stress.

### References

Barrett, M.R. and Lavy, T.L. (1983). J. Environ. Qual., 12 (4), 504-508.
Bestman, H.D.; Devine, M.D. and Vanden Bom, W.H. (1990). Plant Physiol., 93, 1441-1448.
Beyer, E.M.; Duffy, M.J.; Hay, J.V. and Schlueter, D.D. (1988): Sulfonyurea herbicides. In: Herbicides: Chemistry, degradation and mode of action, edited by P.C. Blair, A.M. and Martin, T.C. (1988). Pestic. Sci., 22, 195-219.
Boutin, C., Elmegaard, N. and Kjær, C (1999): Patterns of plant sensitivities to six herbicides with different modes of action. MST-Report (in prep.)
Boutin, C.; Freemark, K.E. and Keddy, C.J. (1993). Technical Report Series. No. 145, 91. Ottawa, Canadian Wildlife Service (headquarters), Environment Canada.
Hamil, A.S.; Marriage, P.B. and Friesen, G. (1977). Weed Sciences, 25, 386-389. Harborne and Baxter, 1995
Jork, H., Funk, W., Fischer, W. and Wimmer, H. (editors) 1988, Dünnschicht-Chromatographie. Band 1a (Weinheim: MERCK, VCH Verlagsgeshellschaft). Kearney et al. 117-189. Marcel Dekker, Inc.
Lydon. J. and Duke, S.O. 1989, Pesticide effects on secondary metabolism of higher plants. Pesticide Science, 25, 361-373.
Moberg, W.K. and Cross, B. (1990). Pestic. Sci., 29, 241-246.
Muir, D.C.G.; Kenny, D.F.; Grift, N.P.; Robinson, R.D.; Titman, R.D. and Murkin, H.R. (1991). Environmental Toxicology and Chemistry, 10, 395-406.
Thomson, W.T. (1987): Agricultural chemicals book II: fungicides. Thomson. Publications, Fresno, CA.
Thomson, W.T. (1989): Agricultural chemicals book II: herbicides*.*
Tomlin, C.D.S. (editor) 2000, Herbicide, The Pesticide Manual. Twelfth Edition, Version 2, British Crop Protection Council, (UK: Binfield, Berks).
Trottier, D.M.; Wong, M.P. and Kent, R.A. (1990): Canadian water quality guidelines for glyphosate. Environment Canada. 170, Ottawa, Ontario.
Ravn, H., (1999): Detection of herbicide spraying in spraying-free areas using plant biomarkers.
Snyder, L. R., (1974): Journal of Chromatography, 92, 223-230.
Stahl, E. (1956): Chemiker-Ztg. 82, 323.
Stahl, E. (1969): Thin-layer chromatography of laboratory handbook. 6th printing, 2nd, 1990, springer, Serlag, Berlin, Heidelberg, New York.
Wagner, H., Bladt, S. and Zgainski, E.M. (editors) 1984, Plant Drug Analysis. A Thin Layer Chromatography Atlas. (Berlin: Springer-Verlag).

### Experimentals

The following is an example of one embodiment of the invention, wherein plant material is tested.

### Method

### Plant material:

The method was tested on 16 different plant species, representing 9 different plant families including both mono- and dicotyledons:

| Plant species | Plant families |
|---|---|
| | |

| Dicotyledons: | |
|---|---|
| *Anagallis arvensis* | Primuláceae |
| *Bellis perennis* | Asteráceae |
| *Inula helenium* | Asteráceae |
| *Rudbekia hirta* | Asteráceae |
| *Centauréa cýanus* | Asteráceae |
| *Leonorus cariaca* | Lamiaceae |
| *Mentha spicata* | Lamiaceae |
| *Nepeta cataria* | Lamiaceae |
| *Prunella vulgaris* | Lamiaceae |
| *Digitalis lanceolata* | Scrophulariaceae |
| *Papaver rhoeas* | Papaveraceae |
| *Fallopia convolvulus* | Polygonaceae |
| *Rumex crispus* | Polygonaceae |
| *Sinapis arvensis* | Brassicaceae |
| *Plantago lanceolata* | Plantagináceae |
| | |

| Monocotyledons: | |
|---|---|
| *Lólium perénne* | Poáceae |
| *Lolium multiflora* | Poáceae |

The plants were sown and cultivated in a green-house and some were grown outdoor (see example 5).

### Green-house conditions:

The experiments were performed in the green-house at the National Environmental Research Institute, Silkeborg, Denmark. All the seeds needed light to germinate, and were sown at the soil surface in individual 11 cm pots. The soil used during the experiments contained: (SM Growing Mould (Growing medium by special soil (sphagnum) added clay granulate), 0-30 mm; grade of turnover 55-75; added per m³: 3 kg lime (white chalk), 1.5 kg dolit chalk, 40 kg clay granulate; Fertilizer added granulate: NO₃-N: 55.0 g; NH₄-N: 65.0 g; P: 106.6 g; K: 224.0 g; Mg: 22 g; Micro nutrients added in chelate form, Mo in uorganic form: B-Boron: 1 g; Cu-copper: 6 g; Mn-Manganese: 3 g; Zinc-Zn: 3 g: Fe-iron: 6 g; Mo- molybdenum: 1.4 g).

During the experiments plants were watered from below, and the temperature in the green-house was maintained between 15 °C and 25 °C with variations due to external weather, and the photoperiod was 16^{th} daylight. The green-house is divided into different units, which allowed for the separate maintenance of the control plants from the sprayed plants to avoid undesirable effects due to vapour drift.

### Outdoor experiments:

The experiments were performed on an experimental plot at the National Environmental Research Institute, Silkeborg, Denmark. The temperature was recorded to calculate degree days after exposure to the herbicide.

### Pesticides (Herbicides) tested:

The above mentioned plants were exposed to the following herbicides:

| Tradename | Active Ingredient | Contents due to the Recommended contents | Additives | Recommended rate |
|---|---|---|---|---|
| Round-up Bio | Glyphosate (GLY) | For analysis: 30% | None | 7.2 g |
| | | The product contains 360 g | | ae⁻¹ ha⁻¹ |
| | | Glyphosate/l, within 480 g | | |
| | | Glyphosate-isopropylaminsalt | | |
| Saxo | Bromoxynil (BRY) | Bromoxynil 20% as octonoate | None | 2 g |
| | | | | ae⁻¹ ha⁻¹ |
| Stomp | Pendimethalin (PEN) | Pendimethalin 400 g/l (36%) | None | 10 g |
| | | | | ae⁻¹ ha⁻¹ |
| Ally | Metsulfuron methyl (METS) | For analysis: Metsulfuron methyl 20% | 0.05% Citowett added to the spray solutions Analysis: Alkylarylpoly-Glykolether 100% | 0.02 g |
| | | | | ae⁻¹ ha⁻¹ |

The herbicides were obtained directly from the producers.

### Glyphosate/Bromoxynil/Pendimethalin/Metsulfuron application:

Glyphosate (Roundup Bio (360 g L⁻¹) was supplied by Monsanto Denmark A/S. Recommended field dose = 1,44 kg a.i. ha⁻¹. In all the experiments, an automatic sprayer (designed in Denmark by Jens Kristensen 1994) was used where the herbicide application is achieved by moving boom equipment with two ordinary hydraulic flat fan nozzles (Hardi 4110-16). Two pots per spray event were placed in the middle of the spray chamber at 50 cm distance from the nozzle, delivering 200 L of water per hectare with a desired herbicide concentration (working pressure 2 bars; speed of spray boom = 4.7 km h⁻¹). The spraying was performed starting with the lowest concentration first, progressing towards the highest concentration. Between each herbicide, the sprayer was thoroughly rinsed several times with water. The control plants were sprayed with water.

### Visual effects on the plants exposed to the herbicides

Four, eight, sixteen and thirty two days after spraying, visual effects on plants were noted before harvest, using the rating chart described in Hamil *et al.* (1977) and Boutin *et al*. (1993); a rating of zero indicates full growth and vigour, and a rating of nine indicates no growth/mortality.

| Rating | Detailed description |
|---|---|
| 0 | No effect |
| 1 | Trace effect: generally associated with slight growth stimulation |
| 2 | Slight effect |
| 3 | Moderate effect: plants 75% the size of control (decrease by 25%) |
| 4 | Injury: plants more than 50% of control and with some clear visible injury on leaves and stems |
| 5 | Definite injury: plants half the size of control, leaf epinasty, plant parts deformed and discoloured |
| 6 | Herbicidial effect: plants 25% size of control, leaf epinasty, plant parts deformed and discoloured |
| 7 | Good herbicidal effect: very small plants, leaf epinasty, plant parts deformed and discoloured |
| 8 | Approaching complete kill, only few green parts left |
| 9 | Complete kill |

### Extraction procedure/sample preparation:

In one aspect of the invention the extraction solvent may be an petroleum-ether extraction solvent, or a 10 % acidic acid in 96 % ethanol extraction solvent. In another aspect the extraction solvent is 75 % ethanol as described below.
Fresh/fresh ethanol extracts/extraction with 75% ethanol:

### • Fresh plant material

The fresh plant material was pressed using a hand press. The extract was immediately centrifuged (3000 rpm) for 10 minutes. The supernatant was used for analysis. The extracts were kept cool or right above 0 °C during the test to avoid decomposition of biomarkers.

### • Frozen plant material

The frozen plant material was pressed using a hand press. The extract was immediately centrifuged (3000 rpm) for 10 minutes. The supernatant was used for analysis. The extracts were kept cool or right above 0 °C during the test to avoid decomposition of biomarkers.

### • Fresh ethanol extract

The fresh plant material was tied with a cotton string and lowered into 96% ethanol for 3 minutes. The plant material was centrifuged slightly manually with a washer (20 rp). The plant material was pressed and centrifuged and kept cool or right above 0 °C as described above. The supernatant was used for analysis and were kept cool during the test to avoid decomposition of biomarkers.

### • Extraction with 75% ethanol

Freeze-dried plant material was crushed and 250 mg was extracted with 5.00 ml 75% ethanol in an ultrasonic bath for 2 hours. The temperature was kept < 0 °C (using ice) during the extraction to avoid decomposition of biomarkers. The extract was centrifuged (3000 rpm) for 10 minutes. The supernatant was used for analysis and were kept cool during the test to avoid decomposition of biomarkers.

Fresh/fresh ethanol extracts/fresh-frozen/extraction with 75% ethanol

### • Fresh plant material

The fresh plant material was pressed using a hand press. The extract was immediately centrifuged (3000 rpm) for 10 minutes. The supernatant was used for analysis. The extracts were kept kept cool or right above 0 °C during the test to avoid decomposition of biomarkers.

### • Fresh ethanol extract

The fresh plant material was tied with a cotton string and lowed into 96% ethanol for 3 minutes. The plant material was centrifuged slightly manually with a washer (20 rp). The plant material was pressed and centrifuged and kept kept cool or right above 0 °C as described above. The supernatant was used for analysis and were kept cool during the test to avoid decomposition of biomarkers.

### • Fresh/frozen

The fresh frozen plant material was pressed using a hand press. The extract was immediately centrifuged (3000 rpm) for 10 minutes. The supernatant was used for analysis. The extracts were kept cool or right above 0 °C during the test to avoid decomposition of biomarkers.

### • Extraction with 75% ethanol

Freeze-dried plant material was crushed and 250 mg was extracted with 5.00 ml 75% ethanol in an ultrasonic bath for 2 hours. The temperature was kept < 0°C (using ice) during the extraction to avoid decomposition of biomarkers. The extract was centrifuged (3000 rpm) for 10 minutes. The supernatant was used for analysis and were kept cool during the test to avoid decomposition of biomarkers.

### Application on TLC-Plates:

The two extracts (fresh/fresh ethanol) were applicated immediately on a TLC plate (10µl on both TLC-Plate types (see below) using cool air-drying. The 75% ethanolic extract was applicated 10µl on Silica-gel TLC-plates and 5µl on Cellulose TLC-plates (see below). The fresh/fresh ethanolic extracts are recommended to perform if fresh plant material are to be analysed (field-test). The freeze-dried or frozen plant material extraction is recommended if the analysis are performed in the laboratory.

### TLC-Systems:

### General definitions for TLC-systems:

Solvent: is placed in the TLC chamber to saturate the chamber with evappurated solvent/eluent prior to placing the TLC-plate with the applied plant extract in the chamber. The solvent initiates the development of the test.
Chemical reagent: is the use of a combination of chemical reagents, wherein the combination of chemical reagents is chosen according to what chemical groups it is desired to detect.
Chemical solvent: is a solvent that is applicated onto the TLC plate to initiate a colour development.

### Non-specific compounds

TLC-System 1: HPTLC-Alufolie Silica Gel 60 F₂₅₄, Merck 1.05548 (10 x 10 cm); 10 µl plant extracts plants exposed to glyphosate and control plants; eluted in n-butanol : acetic acid : water (4:1:5) (upper phase after 5 minutes shaking). Derivatisation: Stock solution 1:50% sulfuric acid in 96% ethanol; Stock solution 2: 2% vanillin in absolute alcohol; Derivatisation-solvent, Fresh produced before use, stock solution 1 and stock solution 2 (1:10); Aftercare: 120°C in 2-3 min. The plate is evaluated visually. The system detects higher alcoholics, phenolic compounds, steroids and etheric oils (Stahl 1969), terpenoids, phenyl propanoids, phenolic compounds (Wagner *et al.* 1984)

TLC-System 7: HPTLC-Alufolie Silica Gel 60 F₂₅₄, Merck 1.05548 (10 x 10 cm);10 µl plant extracts plants exposed to glyphosate and control plants; eluted in n-butanol : acetic acid : water (4:1:5) (upper phase after 5 minutes shaking). Derivatisation: 0,5 g p-anisaldehyde diluted in 10 ml conc. acetic acid and 85 ml absolute alcohol is and 5 ml conc. sulfuric acid (added in the following row). The plate is heated to 10*0°C in 5 to 10 minutes. The plate is evaluated visually or in UV-light at 366 nm. The system detects sugar compounds, steroids, terpens (Stahl, 1969), antioxydants, prostaglandins, phenolic compounds, glycosides, sapogenins, etheric oils, antibiotics, mycotoxins (Jork *et al.,1988*).

### Lipids and terpens

TLC-System 6: HPTLC-Alufolie Silica Gel 60 F₂₅₄, Merck 1.05548 (10 x 10 cm); 10 µl plant extracts plants exposed to glyphosate and control plants; eluted in n-butanol : acetic acid : water (4:1:5) (upper phase after 5 minutes shaking). Derivatisation: 10% molybdatophosphoric acid in 96% ethanol. Treatment after reaction with reagent: The plate is heated at 120°C in few minutes and placed in an ammonium chamber in few seconds until the background of the plate is white. The plate is evaluated visually, white/yellow plate with blue spots. The plate is evaluated visually. The system detects lipids, sterols and steroids (Stahl, 1969), gallus acids, lipid acids, triglycerids, substituted phenolic compounds, indol-derivatives, prostaglandins, etheric oil components, alkaloids (morphine)(Jork *et al.,* 1988).

### Phenolic compounds

TLC-System 2: HPTLC-Alufolie Silica Gel 60 F₂₅₄, Merck 1.05548 (10 x 10 cm); 10 µl plant extracts plants exposed to glyphosate and control plants; eluted in n-butanol : acetic acid : water (4:1:5) (upper phase after 5 minutes shaking). Derivatisation: 1% 2-aminoethyl diphenylborinate in 5% polyethylenglycol 4000 in 96 % ethanol. The last mentioned solution is prepared in ultrasonic bath. The TLC-plate is air-dried and evaluated in UV-light (366 nm). The system detects phenolic compounds, flavonoids, simple phenolic compounds, anthocyanidines; penicillium acid, sugars (Jork *et al.,* 1988) α- og γ-pyrons (Stahl, 1969).

### Amino acids

TLC-System 4d: TLC Alufolie Cellulose, Merck 1.05552 (10 x 10 cm); 5 µl plant extracts plants exposed to glyphosate and control plants; eluted in: n-butanol: 50% formic acid (2:1). Derivatisation: Solvent 1. 0.3 g ninhydrin in 100 ml 2-propanol and 3 ml conc. Acetic acid is added. Solvent 2. Solvent: 0.2% ninhydrin in 96% ethanol. Derivatisation with solvent 1, the plate is air-dried with hot air and derivatisation with solvent 2. Treatment after reaction with reagent: The plate is heated at 110°C in 2 to 3 minutes. The plate is treated with a stabiliser solution (1 ml saturated copper (II) nitrate water solution in 0.2 ml 10% nitric acid and 100 ml absolute alcohol). The plate is visually evaluated, white with red, blue and yellow spots. The system detects amino acids, amines and amino sugar compounds (Stahl, 1969).

### Results:

* Rf-value = The distance of the centre of the spot (i.e. compound) from the start point, divided by the distance of the front of the solvent from the start.

The min. visual effect (VE) of the presence of plant biomarkers is rated as described by Hamil *et al.,* (1977) and Boutin *et al.* (1993).

The transparent shapes with coloured spots at Rf-values* of the plant biomarkers was placed on the TLC-plate. A transparent shape is see-through and was placed on top of the plates for varification of the results. The specific transparent shapes with coloured spots for this specific plant species/herbicide was used. First, the transparent shapes of TLC-System 4d was used, and if the result was positive for one of the used herbicides (based on the mode of action of the herbicides), the other transparent shapes for the other TLC-Systems (1, 2, 5, 8, 11, 16 and 21) were used to verify the result. If the plant species was not identified, the most frequent transparent shapes were used.

The most frequent shapes were developed by evaluating the responses (biomarkers) in all situations from all the tested plants, and where at least 5 plant species out of the 10 plant species resulted in the same response, a common biomarker was established.

As described below, tables and transparent shapes were performed for four herbicides having different modes of action. The pattern of the Rf-values of the plant biomarkers present in the plants after exposure to the four different herbicides, Glyphosate, Metsulfuron methyl, Pendimethalin and Bromoxynil was found to be different. If the plant species has been exposed to one of the herbicides, the plant biomarkers were present. Most of the plant biomarkers were present even if the visual effect was 0 and 1% of recommended label dose.

The visual effect of the tested herbicides were not present in all tested plants, but the finger-print of compounds were identified (see examples below). However, even if the visual effects were not present on the plants, the plant biomarkers were present in most of the tested plants.

### Example 1:

### Anagallis arvensis (Primuláceae)

The plant species: *Anagallis arvensis* (Primuláceae) was contaminated with Glyphosate, Pendimethalin, Bromoxynil and Metsulfuron methyl. The plant biomarker responses were as follows: (4 days - 60 degree days, 8 days - 120 degree days, 14 days - 180 degree days, 16 days - 240 degree days, 32 days - 480 degree days)

### Glyphosate, TLC 4d

| Rf-value | Colour | Days after exposure | Min. of label dose of herbicide with effect | Min. visual effect |
|---|---|---|---|---|
| 0.11 | Violet | 8,14,16,32 | 1% | 1 |
| 0.14* | Red | 8,16 | 1% | 1 |
| 0.41 | Yellow | 4,8,14,16,32 | 1% | 0 |
| 0.53 | Violet | 4,8,14,16,32 | 1% | 0 |
| 0.58 | Red | 8,14,16 | 1% | 0 |
| 0.65 | Violet | 4,8,14,16,32 | 1% | 0 |
| 0.73 | Rød | 4,8,14,16 | 1% | 0 |

### Glyphosate, TLC 1

| Rf-value | Colour | Days after exposure | Min. of label dose of herbicide with effect | Min. visual effect |
|---|---|---|---|---|
| 0.08 | Blue green | 4,8,14,16,32 | 1% | 0 |
| 0.32 | Red violet | 8,14,32 | 1% | 3 |
| 0.34 | Blue | 8,32 | 1% | 1 |
| 0.59* | Blue | 8,16,32 | 1% | 1 |
| 0.77* | Grey blue | 8,16,32 | 1% | 1 |

### Glyphosate, TLC 2

| Rf-value | Colour UV-light 366nm | Days after exposure | Min. of label dose of herbicide with effect | Min. visual effect |
|---|---|---|---|---|
| 0.06* | Blue | 8,16 | 1% | 1 |
| 0.44* | Blue | 16,32 | 10% | 7 |
| 0.58 | Blue | 4,8,14,16,32 | 1% | 0 |
| 0.72 | Blue | 4,8,16 | 1% | 0 |
| 0.89 | Blue | 8,16,32 | 10% | 3 |
| 0.90* | Blue | 16,32 | 1% | 3 |

### Glyphosate, TLC 6

| Rf-value | Colour | Days after exposure | Min. of label dose of herbicide with effect | Min. visual effect |
|---|---|---|---|---|
| 0.07 | Blue | 8,14,16,32 | 1% | 1 |
| 0.27 | Blue | 8,14,32 | 1% | 1 |
| 0.56* | Blue | 4,8 | 1% | 0 |
| 0.80* | Blue | 4,8,16,32 | 1% | 0 |

### Glyphosate, TLC 7

| Rf-value | Colour | Days after exposure | Min. of label dose of herbicide with effect | Min. visual effect |
|---|---|---|---|---|
| 0.08 | Grey | 4,8,14,16,32 | 1% | 0 |
| 0.33 | Blue | 8,14,32 | 1% | 3 |
| 0.33 | Red | 14,32 | 1% | 1 |
| 0.49* | Blue grey | 8,16 | 1% | 1 |
| 0.61** | Yellow | 4,8,16,32 | 10% | 0 |
| 0.83* | Grey | 8,16,32 | 1% | 1 |

| | | | | |
|---|---|---|---|---|
| * Only present for fresh extracts ** Very good biomarker | | | | |

### Pendimethalin, TLC 4d

| Rf-value | Colour | Days after exposure | Min. of label dose of herbicide with effect | Min. visual effect |
|---|---|---|---|---|
| 0.12 | Violet | 4,8,14,16,32 | 1% | 0 |
| 0.40* | Violet | 8,16 | 1% | 1 |
| 0.51 | Violet | 4,8,16,32 | 1% | 0 |
| 0.56 | Red | 14,16 | 1% | 1 |
| 0.65 | Violet | 4,8,14,16,32 | 1% | 0 |

### Pendimethalin, TLC 1

| Rf-value | Colour | Days after exposure | Min. of label dose of herbicide with effect | Min. visual effect |
|---|---|---|---|---|
| 0.20 | Red violet | 8,14,32 | 10% | 1 |
| 0.31 | Red violet | 4,14,32 | 10% | 0 |

### Pendimethalin, TLC 2

| Rf-value | Colour UV-light 366nm | Days after exposure | Min. of label dose of herbicide with effect | Min. visual effect |
|---|---|---|---|---|
| 0.52 | Cobalt blue | 4,16 | 10% | 0 |
| 0.90** | Yellow | 8,32 | 1% | 1 |

### Pendimethalin, TLC 7

| Rf-value | Colour | Days after exposure | Min. of label dose of herbicide with effect | Min. visual effect |
|---|---|---|---|---|
| 0.32 | Red violet | 14,32 | 10% | 3 |

| | | | | |
|---|---|---|---|---|
| * Only present for fresh extracts ** Only present for extraction with 75% ethanol | | | | |

### Bromoxynil, TLC 4d

| Rf-value | Colour | Days after exposure | Min. of label dose of herbicide with effect | Min. visual effect |
|---|---|---|---|---|
| 0.10 | Violet | 8,4 | 10% | 0 |
| 0.26 | Red violet | 8,14,32 | 10% | 0 |
| 0.56 | Violet | 8,14,16 | 1% | 0 |
| 0.74 | Red | 4,8,14 | 1% | 0 |

### Bromoxynil, TLC 6

| Rf-value | Colour | Days after exposure | Min. of label dose of herbicide with effect | Min. visual effect |
|---|---|---|---|---|
| 0.33 | Blue | 8,14,32 | 1% | 0 |
| 0.84 | Blue | 8,32 | 1% | 0 |

### Bromoxynil, TLC 7

| Rf-value | Colour | Days after exposure | Min. of label dose of herbicide with effect | Min. visual effect |
|---|---|---|---|---|
| 0.33 | Red | 8,14,32 | 1% | 0 |
| 0.35 | Blue | 8,14,32 | 1% | 0 |
| 0.85* | Blue | 4,8,32 | 1% | 0 |

| | | | | |
|---|---|---|---|---|
| * Only present for fresh extracts | | | | |

### Metsulfuron methyl, TLC 4d

| Rf-value | Colour | Days after exposure | Min. of label dose of herbicide with effect | Min. visual effect |
|---|---|---|---|---|
| 0.14 | Violet | 4,8,14,16,32 | 1% | 0 |
| 0.39 | Yellow | 14,16,32 | 1% | 1 |
| 0.46* | Violet | 8,16 | 10% | 3 |
| 0.53 | Violet | 8,14,16,32 | 1% | 1 |
| 0.70 | Violet | 4,8,14 | 1% | 0 |

### Metsulfuron methyl, TLC 1

| Rf-value | Colour | Days after exposure | Min. of label dose of herbicide with effect | Min. visual effect |
|---|---|---|---|---|
| 0.07 | Blue green | 8,14,16,32 | 1% | 1 |
| 0.75* | Grey | 4,8,32 | 10% | 0 |
| 0.93 | Red violet | 8,16,32 | 1% | 1 |

### Metsulfuron methyl, TLC 2

| Rf-value | Colour UV-light 366nm | Days after exposure | Min. of label dose of herbicide with effect | Min. visual effect |
|---|---|---|---|---|
| 0.05* | Blue | 8,16 | 1% | 1 |
| 0.68 | Blue | 8,16,32 | 1% | 1 |
| 0.90 | Yellow | 8,16,32 | 1% | 1 |
| 0.95 | Blue | 16,32 | 10% | 7 |

### Metsulfuron methyl, TLC 6

| Rf-value | Colour | Days after exposure | Min. of label dose of herbicide with effect | Min. visual effect |
|---|---|---|---|---|
| 0.09 | Blue | 4,8,14,16 | 1% | 0 |
| 0.30 | Blue | 14,32 | 10% | 8 |
| 0.83* | Blue | 8,16,32 | 10% | 3 |
| 0.93* | Blue | 8,16,32 | 1% | 3 |

### Metsulfuron methyl, TLC 7

| Rf-value | Colour | Days after exposure | Min. of label dose of herbicide with effect | Min. visual effect |
|---|---|---|---|---|
| 0.07 | Green | 8,16 | 1% | 1 |
| 0.77* | Green blue | 8,16,32 | 1% | 1 |
| 0.93 | Red violet | 8,16,32 | 1% | 1 |

| | | | | |
|---|---|---|---|---|
| *Only present for fresh extracts | | | | |

### Example 2:

### Centauréa cýanus L., (Asteráceae)

The plant species: *Centauréa cýanus L.*, (Asteráceae) was contaminated with Glyphosate, Pendimethalin, Bromoxynil and Metsulfuron methyl. The plant biomarker responses were as follows:

### Pendimethalin, TLC 4d

| Rf-value | Colour | Days after exposure | Min. of label dose of herbicide with effect | Min. visual effect |
|---|---|---|---|---|
| 0.11* | Violet | 4,16 | 1% | 0 |
| 0.60* | Violet | 4,16,32 | 1% | 0 |

### Pendimethalin, TLC 2

| Rf-value | Colour UV-light 366nm | Days after exposure | Min. of label dose of herbicide with effect | Min. visual effect |
|---|---|---|---|---|
| 0.44 | Orange/Y ellow | 4,8,16 | 1% | 0 |

| | | | | |
|---|---|---|---|---|
| * Only present for fresh extracts | | | | |

### Bromoxynil, TLC 4d

| Rf-value | Colour | Days after exposure | Min. of label dose of herbicide with effect | Min. visual effect |
|---|---|---|---|---|
| 0.12 | Violet | 4,14,32 | 1% | 0 |
| 0.52* | Violet | 4,8,16 | 10% | 1 |
| 0.62 | Red | 4,8,14,16 | 1% | 0 |
| 0.66 | Violet | 4,8,14,16,32 | 1% | 0 |
| 0.76 | Red | 4,8,14 | 1% | 0 |
| 0.39 | Gul | 14,16,32 | 1% | 1 |

### Bromoxynil, TLC 2

| Rf-value | Colour | Days after exposure | Min. of label dose of herbicide with effect | Min. visual effect |
|---|---|---|---|---|
| 0.49 | Cobalt blue | 4,8,16 | 10% | 1 |

### Bromoxynil, TLC 7

| Rf-value | Colour | Days after exposure | Min. of label dose of herbicide with effect | Min. visual effect |
|---|---|---|---|---|
| 0.09 | Green blue | 4,8,32 | 1% | 0 |

| | | | | |
|---|---|---|---|---|
| * Only present for fresh extracts | | | | |

### Metsulfuron methyl, TLC 4d

| Rf-value | Colour | Days after exposure | Min. of label dose of herbicide with effect | Min. visual effect |
|---|---|---|---|---|
| 0.15 | Violet | 8,14,16 | 1% | 1 |
| 0.41 | Yellow | 14,32 | 1% | 0 |
| 0.59 | Violet | 8,14 | 1% | 0 |
| 0.65 | Red | 8,14 | 1% | 0 |
| 0.68 | Violet | 4,8,32 | 1% | 0 |

### Metsulfuron methyl, TLC 2

| Rf-value | Colour UV-light 366nm | Days after exposure | Min. of label dose of herbicide with effect | Min. visual effect |
|---|---|---|---|---|
| 0.52 | Yellow/Or ange | 14,16 | 1% | 1 |
| 0.86 | Blue | 4,16,32 | 1% | 0 |

### Metsulfuron methyl, TLC 6

| Ref-values | Colour | Days after exposure | Min. of label dose of herbicide with effect | Min. visual effect |
|---|---|---|---|---|
| 0,46 | Blue | 16,32 | 1% | 0 |

### Metsulfuron methyl, TLC 7

| Rf-value | Colour | Days after exposure | Min. of label dose of herbicide with effect | Min. visual effect |
|---|---|---|---|---|
| 0.42 | Blue | 14,16,32 | 1% | 0 |

| | | | | |
|---|---|---|---|---|
| * Only present for fresh extracts | | | | |

### Glyphosate, TLC 4d

| Rf-value | Colour | Days after exposure | Min. of label dose of herbicide with effect | Min. visual effect |
|---|---|---|---|---|
| 0.13 | Violet | 8,14,16,32 | 1% | 0 |
| 0.26 | Red | 4,8,14,16 | 10% | 3 |
| 0.42 | Yellow | 14,16,32 | 1% | 0 |
| 0.48 | Red | 4,8,16 | 1% | 1 |
| 0.52 | Violet | 4,8,14,32 | 1% | 0 |
| 0.63 | Red | 4,8,14,16,32 | 1% | 0 |
| 0.67 | Violet | 8,14,16,32 | 1% | 0 |
| 0.73 | Red | 4,8,14,16,32 | 1% | 0 |

### Glyphosate, TLC 1

| Rf-value | Colour UV-light 366nm | Days after exposure | Min. of label dose of herbicide with effect | Min. visual effect |
|---|---|---|---|---|
| 0.10 | Blue green | 4,8,16,32 | 10% | 3 |
| 0.66*** | Blue | 4,8,16,32 | 10% | 3 |

### Glyphosate, TLC 2

| Rf-value | Colour | Days after exposure | Min. of label dose of herbicide with effect | Min. visual effect |
|---|---|---|---|---|
| 0.18 | Blue | 16,32 | 10% | 5 |
| 0.51 | Cobalt blue | 4,16,32 | 1% | 1 |
| 0.66 | Cobalt blue | 4,8,16,32 | 1% | 0 |
| 0.85 | Blue | 4,8,16,32 | 1% | 1 |
| 0.94 | Cobalt blue | 14,16,32 | 10% | 5 |

### Glyphosate, TLC 6

| Rf-value | Colour | Days after exposure | Min. of label dose of herbicide with effect | Min. visual effect |
|---|---|---|---|---|
| 0.10 | Blue | 4,8,16 | 10% | 3 |

### Glyphosate, TLC 7

| Rf-value | Colour | Days after exposure | Min. of label dose of herbicide with effect | Min. visual effect |
|---|---|---|---|---|
| 0.08 | Green blue | 4,8,16,32 | 10% | 3 |

| | | | | |
|---|---|---|---|---|
| * Only present for fresh extracts *** Very good biomarker | | | | |

### Example 3:

### Lólium perénne L., (Poáceae)

The plant species: *Lólium perénne L.*, (Poáceae) was contaminated with Glyphosate, Pendimethalin, Bromoxynil and Metsulfuron methyl. The plant biomarker responses were as follows:

### Pendimethalin, TLC 4d

| Rf-value | Colour | Days after exposure | Min. of label dose of herbicide with effect | Min. visual effect |
|---|---|---|---|---|
| 0.14 | Violet | 4,8,16,32 | 1% | 0 |

### Pendimethalin, TLC 7

| Rf-value | Colour | Days after exposure | Min. of label dose of herbicide with effect | Min. visual effect |
|---|---|---|---|---|
| 0.02 | Green | 8,16 | 1% | 0 |

### Bromoxynil, TLC 4d

| Rf-value | Colour | Days after exposure | Min. of label dose of herbicide with effect | Min. visual effect |
|---|---|---|---|---|
| 0.15* | Violet | 4,32 | 10% | 0 |
| 0.39 | Gul | 14,16,32 | 1% | 1 |

### Bromoxynil, TLC 1

| Rf-value | Colour | Days after exposure | Min. of label dose of herbicide with effect | Min. visual effect |
|---|---|---|---|---|
| 0.03 | Green blue | 8,16,32 | 1% | 0 |

### Bromoxynil, TLC 6

| Rf-value | Colour | Days after exposure | Min. of label dose of herbicide with effect | Min. visual effect |
|---|---|---|---|---|
| 0.56 | Blue | 8,32 | 10% | 0 |

| | | | | |
|---|---|---|---|---|
| * Only for fresh extracts | | | | |

### Metsulfuron methyl, TLC 4d

| Ref-value | Colour | Days after exposure | Min. of label dose of herbicide with effect | Min. visual effect |
|---|---|---|---|---|
| 0.15 | Violet | 4,8,16,32 | 1% | 0 |
| 0.16* | Red | 8,16 | 10% | 0 |
| 0.39 | Violet | 8,16,32 | 10% | 0 |
| 0.51 | Violet | 4,8,16,32 | 10% | 0 |

### Metsulfuron methyl, TLC 1

| Rf-value | Colour UV-light 366nm | Days after exposure | Min. of label dose of herbicide with effect | Min. visual effect |
|---|---|---|---|---|
| 0.05 | Green | 4,8,16,32 | 10% | 0 |
| 0.19* | Green | 8,16 | 1% | 0 |

### Metsulfuron methyl, TLC 2

| Rf-value | Colour | Days after exposure | Min. of label dose of herbicide with effect | Min. visual effect |
|---|---|---|---|---|
| 0.06* | Blue | 16,32 | 1% | 0 |
| 0.29* | Blue | 8,16 | 10% | 0 |
| 0.49 | Cobalt blue | 4,8,16,32 | 1% | 0 |
| 0.73 | Blue | 16,32 | 10% | 2 |

### Metsulfuron methyl, TLC 6

| Rf-value | Colour | Days after exposure | Min. of label dose of herbicide with effect | Min. visual effect |
|---|---|---|---|---|
| 0.06 | Blue | 4,8,16,32 | 10% | 0 |
| 0.50* | Blue | 4,16,32 | 10% | 0 |

### Metsulfuron methyl, TLC 7

| Rf-value | Colour | Days after exposure | Min. of label dose of herbicide with effect | Min. visual effect |
|---|---|---|---|---|
| 0.05 | Green | 8,16,32 | 10% | 0 |
| 0.13* | Green | 8,16 | 10% | 0 |

| | | | | |
|---|---|---|---|---|
| * Only for fresh extracts | | | | |

### Glyphosate, TLC 4d

| Rf-value | Colour | Days after exposure | Min. of label dose of herbicide with effect | Min. visual effect |
|---|---|---|---|---|
| 0.13 | Violet | 4,8,11,16,32 | 1% | 0 |
| 0.18 | Red | 8,11,16,32 | 1% | 2 |
| 0.39 | Yellow | 11,16,32 | 10% | 2 |
| 0.46 | Violet | 4,8,16,32 | 10% | 0 |
| 0.54 | Violet | 11,16,32 | 10% | 5 |
| 0.60 | Red | 8,11,16,32 | 10% | 5 |
| 0.65 | Violet | 11,16,32 | 10% | 5 |
| 0.73 | Red | 16,32 | 10% | 5 |

### Glyphosate, TLC 1

| Rf-value | Colour | Days after exposure | Min. of label dose of herbicide with effect | Min. visual effect |
|---|---|---|---|---|
| 0.00 | Green blue | 4,11,16 | 10% | 0 |
| 0.08 | Blue green | 4,8,11,16,32 | 1% | 0 |
| 0.50 | Yellow | 11,32 | 10% | 7 |
| 0.53 | Blue | 8,16,32 | 10% | 0 |
| 0.94 | Blue green | 4,11,16,32 | 10% | 0 |

### Glyphosate, TLC 2

| Rf-value | Colour UV-light 366nm | Days after exposure | Min. of label dose of herbicide with effect | Min. visual effect |
|---|---|---|---|---|
| 0.15 | Orange | 16,32 | 10% | 5 |
| 0.47 | Cobalt blue | 11,16,32 | 1% | 2 |
| 0.63 | Coblat blue | 4,8,32 | 1% | 0 |
| 0.76 | Blue | 16,32 | 1% | 2 |

### Glyphosate, TLC 6

| Rf-value | Colour | Days after exposure | Min. of label dose of herbicide with effect | Min. visual effect |
|---|---|---|---|---|
| 0.07 | Blue | 4,8,11,16,32 | 1% | 0 |
| 0.21 | Blue | 4,8,16,32 | 10% | 0 |
| 0.49 | Blue | 16,32 | 10% | 5 |
| 0.94 | Blue | 4,11,32 | 10% | 0 |

### Glyphosate, TLC 7

| Rf-value | Colour | Days after exposure | Min. of label dose of herbicide with effect | Min. visual effect |
|---|---|---|---|---|
| 0.00 | Blue green | 8,16,32 | 1% | 2 |
| 0.07 | Grey | 4,8,11,16,32 | 1% | 0 |
| 0.48** | Red violet | 11,16,32 | 10% | 5 |

| | | | | |
|---|---|---|---|---|
| * Only for fresh extracts *** Very good biomarker | | | | |

### Example 4:

### P/antágo lanceoláta L., (Plantagináceae)

The plant species *Plantágo lanceoláta L.*, (Plantagináceae) was contaminated with Glyphosate, Pendimethalin, Bromoxynil and Metsulfuron methyl. The plant biomarker responses were as follows:

### Pendimethalin, TLC 4d

| Rf-value | Colour | Days after exposure | Min. of label dose of herbicide with effect | Min. visual effect |
|---|---|---|---|---|
| 0.15 | Violet | 4,8,16 | 1% | 0 |

### Pendimethalin, TLC 2

| Rf-value | Colour UV-light 366nm | Days after exposure | Min. of label dose of herbicide with effect | Min. visual effect |
|---|---|---|---|---|
| 0.76* | Blue | 16,32 | 1% | 0 |

### Pendimethalin, TLC 6

| Rf-value | Colour | Days after exposure | Min. of label dose of herbicide with effect | Min. visual effect |
|---|---|---|---|---|
| 0.74* | Blue | 16,32 | 1% | 0 |

| | | | | |
|---|---|---|---|---|
| * Only present for fresh extracts ** Only present for extraction with 75% ethanol | | | | |

### Bromoxynil, TLC 4d

| Rf-value | Colour | Days after exposure | Min. of label dose of herbicide with effect | Min. visual effect |
|---|---|---|---|---|
| 0.14 | Violet | 16,32 | 1% | 0 |
| 0.28** | Red | 4,8 | 10% | 0 |
| 0.53** | Red | 16,32 | 10% | 2 |
| 0.64 | Violet | 16,32 | 10% | 2 |
| 0.74 | Red | 4,8,14 | 1% | 0 |

| | | | | |
|---|---|---|---|---|
| ** Only present for extraction with 75% ethanol | | | | |

### Metsulfuron methyl, TLC 4d

| Rf-value | Colour | Days after exposure | Min. of label dose of herbicide with effect | Min. visual effect |
|---|---|---|---|---|
| 0.16 | Violet | 8,16,32 | 1% | 0 |
| 0.28** | Red | 4,8 | 1% | 1 |
| 0.73* | Red | 16,32 | 1% | 1 |

### Metsulfuron methyl, TLC 1

| Rf-value | Colour | Days after exposure | Min. of label dose of herbicide with effect | Min. visual effect |
|---|---|---|---|---|
| 0.08 | Green | 4,8,16,32 | 1% | 0 |

### Metsulfuron methyl, TLC 2

| Rf-value | Colour UV-light 366nm | Days after exposure | Min. of label dose of herbicide with effect | Min. visual effect |
|---|---|---|---|---|
| 0.07* | Blue | 4,8,16,32 | 1% | 0 |
| 0.38* | Blue | 4,8,16 | 1% | 0 |
| 0.74* | Blue | 4,8,16 | 1% | 0 |

### Metsulfuron methyl, TLC 6

| Rf-value | Colour | Days after exposure | Min. of label dose of herbicide with effect | Min. visual effect |
|---|---|---|---|---|
| 0.05 | Blue | 4,8,14,16 | 10% | 2 |

### Metsulfuron methyl, TLC 7

| Rf-value | Colour | Days after exposure | Min. of label dose of herbicide with effect | Min. visual effect |
|---|---|---|---|---|
| 0.06 | Olive green | 8,16,32 | 10% | 3 |

| | | | | |
|---|---|---|---|---|
| * Only present for fresh extracts ** Only present for extraction with 75% ethanol | | | | |

### Glyphosate, TLC 4d

| Rf-value | Colour | Days after exposure | Min. of label dose of herbicide with effect | Min. visual effect |
|---|---|---|---|---|
| 0.13 | Violet | 4,8,16,32 | 1% | 0 |
| 0.26* | Red | 4,8,16,32 | 1% | 3 |
| 0.44 | Violet | 16,32 | 1% | 7 |
| 0.52 | Violet | 16,32 | 1% | 4 6 |
| 0.60 | Red | 8,16,32 | 1% | 0 |
| 0.68 | Violet | 8,16,32 | 1% | 0 |
| 0.73 | Red | 16,32 | 1% | 4 6 |

### Glyphosate, TLC 1

| Rf-value | Colour | Days after exposure | Min. of label dose of herbicide with effect | Min. visual effect |
|---|---|---|---|---|
| 0.07 | Olive green | 8,16,32 | 1% | 0 |

### Glyphosate, TLC 2

| Rf-value | Colour UV-light 366nm | Days after exposure | Min. of label dose of herbicide with effect | Min. visual effect |
|---|---|---|---|---|
| 0.07 | Blue | 8,16,32 | 1% | 0 |
| 0.18 | Orange | 8,16,32 | 1% | 0 |
| 0.19* | Blue | 4,8,16 | 1% | 0 |
| 0.35* | Blue | 4,8,16 | 1% | 0 |
| 0.51* | Cobalt blue | 8,16,32 | 1% | 4 |
| 0.62 | Blue | 4,8,16,32 | 1% | 1 |

### Glyphosate, TLC 6

| Rf-value | Colour | Days after exposure | Min. of label dose of herbicide with effect | Min. visual effect |
|---|---|---|---|---|
| 0.07 | Blue | 16,32 | 1% | 4 |

### Glyphosate, TLC 7

| Rf-value | Colour | Days after exposure | Min. of label dose of herbicide with effect | Min. visual effect |
|---|---|---|---|---|
| 0.05 | Green | 16,32 | 1% | 4 |
| 0.12 | Blue green | 8,16,32 | 1% | 0 |

| | | | | |
|---|---|---|---|---|
| * Only present for fresh extracts | | | | |

### Example 5:

The following are the most frequent biomarker pattern responses after exposure to all the above herbicides:

### Pendimethalin, TLC 4d (amino acids)

| Rf-value | Colour |
|---|---|
| 0.14 | Violet |
| 0.61 | Violet |

### Bromoxynil, TLC 4d (amino acids)

| Rf-value | Colour |
|---|---|
| 0.14 | Violet |
| 0.64 | Violet |
| 0.73 | Red |

### Metsulfuron methyl, TLC 4d (amino acids)

| Rf-value | Colour |
|---|---|
| 0.15 | Violet |
| 0.43 | Yellow |
| 0.53 | Violet |
| 0.66 | Violet |
| 0.73 | Red |

### Glyphosate, TLC 4d (amino acids)

| Rf-value | Colour |
|---|---|
| 0.14 | Violet |
| 0.43 | Yellow |
| 0.52 | Violet |
| 0.60 | Red |
| 0.64 | Violet |
| 0.73 | Red |

### Metsulfuron methyl, TLC 1 (non-specific compounds)

| Rf-value | Colour |
|---|---|
| 0.06 | Green blue |
| 0.23 | Green blue |
| 0.73 | Grey |

### Glyphosate, TLC 1 (non-specific compounds)

| Rf-value | Colour |
|---|---|
| 0.08 | Green blue |
| 0.60 | Blue |

### Pendimethalin, TLC 2 (Phenolic compounds)

| Rf-value | Colour |
|---|---|
| 0.45 | Yellow |

### Metsulfuron methyl, TLC 2 (Phenolic compounds)

| Rf-value | Colour |
|---|---|
| 0.06 | Blue |
| 0.44 | Blue |
| 0.72 | Blue |

### Glyphosate, TLC 2 (Phenolic compounds)

| Rf-value | Colour |
|---|---|
| 0.50 | Cobalt blue |
| 0.68 | Blue |
| 0.85 | Blue |

### Metsulfuron methyl, TLC 6 (Lipids and Terpens)

| Rf-value | Colour |
|---|---|
| 0.06 | Blue |
| 0.44 | Blue |
| 0.92 | Blue |

### Glyphosate, TLC 6 (Lipids and Terpens)

| Rf-value | Colour |
|---|---|
| 0.08 | Blue |

### Metsulfuron methyl, TLC 7 (non-specific compounds)

| Rf-value | Colour |
|---|---|
| 0.08 | Green blue |

### Glyphosate, TLC 7 (non-specific compounds)

| Rf-value | Colour |
|---|---|
| 0.06 | Green |

### Example 6:

The following example illustrates one embodiment of the invention wherein glyphosate is applied to different plant species grown indoors and outdoors. The embodiment also concerns sensitivity and stability of the biomarkers

### Glyphosate application:

Glyphosate (Roundup Bio (360 g L⁻¹) was supplied by Monsanto Denmark A/S. Recommended field dose = 1,44 kg a.i. ha⁻¹. In all the experiments, an automatic sprayer (designed in Denmark by Jens Kristensen 1994) was used where the herbicide application is achieved by moving boom equipment with two ordinary hydraulic flat fan nozzles (Hardi 4110-16). Two pots per spray event were placed in the middle of the spray chamber at 50 cm distance from the nozzle, delivering 200 L of water per hectare with a desired herbicide concentration (working pressure 2 bars; speed of spray boom = 4.7 km h⁻¹). The spraying was performed starting with the lowest concentration first, progressing towards the highest concentration. Between each herbicide, the sprayer was thoroughly rinsed several times with water. The control plants were sprayed with water.

### Experiments with different plant species:

The study was performed in greenhouse between August and December 1998 for all species. Degree days (= (Present temperature in °C - 5°C) x amount of days) were used as time scale. The plants were 226-378 degree days (30 days) old before exposure. The plants were exposed to 0, 1, 10 and 100% of the recommended field dose of Roundup Bio. Before the harvest, the plants were evaluated for visual effects using the rating chart described in Table 1 (Hamil *et al.,* 1977). A rating of zero indicates full growth and vigour (compared with control plants) and a rating of nine, no growth/mortality. The plants were all harvested 180 degree days (14 days) after exposure. The plants were lyophilizied and kept dry and in dark before phytochemical analysis.

### Stability experiments

Investigations of the phytochemical changes in relation to time were performed in greenhouse between January and March 2000 for the group of plant species, Ana*gallis arvensis*, *Centauréa cýanus*, *Lólium perénne*, and *Plantágo lanceoláta*. The plants were 891 degree days (39 days) old before exposure. The plants were exposed to 0, 1, 10 and 100% of the recommended field dose of Roundup Bio. Before harvest, the visual effect on the plants was evaluated as described above. The plants were harvested 60 degree days (4 days), 120 degree days (8 days), 240 degree days (16 days), and 480 degree days (32 days) after exposure.

### Sensitivity in relation to plant age

To investigate the presence of phytochemical changes and the sensitivity of plants in relation to age, two plant species, *A*. *arvensis* and *C*. *cyanus* were tested January and February 2001. The plants were sown displaced and all plants with different ages were exposed at the same time. The plants were 121 degree days (7 days) (only *C*. *cyanus),* 208 degree days (14 days), 316 degree days (21 days), 426 degree days (28 days) and 533 degree days (35 days) before exposure. The plants were exposed to 0, 1 and 10% of recommended field dose of Roundup Bio. Before harvest, the visual effect on the plants was evaluated as described above. The plants were harvested 61 degree days (4 days), 119 degree days (8 days) and 245 degree days (17 days) after exposure.

### Comparison of greenhouse and outdoor experiments

A comparison study was performed to compare the results of the above mentioned greenhouse experiments with the results of outdoor experiment. The experiment was performed between June and July 2000 using the most sensitive plant species, *A*. *arvensis* and *L. perenne*. Degree days were calculated as described above. The plants were 77 days (670 degree days) old before exposure. The plants were exposed to 0, 1, 10 and 100% of the recommended field dose of glyphosate. Before harvest, the visual effect on the plants was evaluated as described above. The plants were harvested 32 degree days (4 days), 61 degree days (7 days), 139 degree days (17 days) and 242 degree days (27 days) after exposure.

### Plant extraction and treatment

250 mg lyophilized plant material was crushed and extracted with 5.00 ml 75 % ethanol in water in ultrasonic bath for two hours. Ice was added to the bath every 30 minutes to keep the temperature constant during the extraction. The extracts were transferred to vials and centrifuged (3000g per min.) for 10 minutes. The extract was immediately used for phytochemical analysis.

Frozen or fresh plant material was pressed for plant sap using a hand press. The sap was transferred to vials and centrifuged (3000g per min.) for 10 minutes. The extract was immediately used for phytochemical analysis.

### Thin Layer Chromatography (TLC) analysis

Rf-values (defined as, Rf-value = Distance of spot centre from start point / Distance of solvent front from start point (Stahl, 1969)) were detemined for all phytochemical compounds.

### Results

### Plant extraction and treatment

There was no difference in the results obtained with fresh or frozen pressed sap. The plant extracts of lyophilised plant material were compared with fresh and frozen sap. Less plant biomarkers were present in the analysed extracts of lyophilised plant material than in the fresh or frozen plant sap. Therefore only frozen pressed sap was used for analysis.

### Comparison of TLC-systems

More than 25 different TLC-systems (type of TLC-plate; solvent and derivatisation reagent) were tested to find the most valuable systems to detect phytochemical changes in exposed plant extracts. The phytochemical changes in exposed plants compared with control plants were detected visually using all five TLC systems. For each plant species and TLC-system a characteristic pattern of spots was identified in comparison of treated and un-treated plants, including missing and new compounds. In this study only the pattern of new compounds in exposed plants was used. To evaluate the pattern of different groups of compounds, TLC-systems 1, 6 and 7 was used. Two specific TLC-systems (4d and 2) were developed to highlight the pattern of amino acids and phenolic compounds, both of which are important to the physiological mechanism of glyphosate (Tomlin *et al.* 2000; Lydon & Duke 1989).

### Screening for phytochemical changes

Plant extracts screened for phytochemical changes in all five TLC-systems showed different patterns for each plant species. In Table 3, two of the most sensitive plant species, *Anagallis arvensis* and *Lólium perénne* are presented to illustrate the different pattern of phytochemical compounds in plants exposed to Roundup Bio. The most frequent pattern of "new" developed compounds (three amino acids, two general compounds, four phenolic compounds) was seen in more than 50% of all tested plants species. 81% of all tested plant species had a "new" lipid/terpen in exposed plants. These compounds are marked with * in Table 3

### Stability

Phytochemical changes were seen 4 days (60 degree days) after exposure and a phytochemical pattern was present up to 32 days (480 degree days) after exposure. Due to high sensitivity, young plants showed lower stability of the biochemical changes.

### Sensitivity

In table 2 the sensitivity of the different plant species to Roundup Bio is presented. The sensitivity of the plant species was evaluated on basis of the presence of the most frequent pattern, the lowest concentration of exposure, and the visual effect of the exposed plants. In table 2, the plant species are divided into three groups: Group I: Very sensitive plant species; Group II: Medium sensitive plant species, and Group III: Less sensitive plant species. In Group I, a phytochemical pattern was present in plants exposed to 1 % of the recommended field dose of Roundup Bio with a visual effect from 0 to 4. In Group II, a phytochemical pattern was found in plants exposed to 1 to 10% of the recommended field dose of the herbicide with a visual effect from 0 to 5. Finally, Group III presents plant species containing a phytochemical pattern after exposure of 10% of the recommended field dose with a visual effect from 5 to 7.

All the plant species representing the families: Primuliceae, Poáceae Polygonaceae, Plantagináceae and Papaveraceae were found in the group of very sensitive plant species. In all three groups, no specific trend was found for plant species of Asteráceae. The plant species representing Lamiaceae were found in the group of medium and less sensitive plant species. *D. lanceolata* (Scrophulariaceae) was medium and *S*. *arvensis* (Brassicaceae) was less sensitive. *A. arvensis* and *L. perenne* were the most sensitive plant species in the present study.

The correlation between plant age and sensitivity was studied for A. *arvensis* and *C*. *cyanus.* The sensitivity for both *A. arvensis* and *C*. *cyanus* were alike for young and older plants, based on the phytochemical pattern at low exposure, 1% and 10% of the recommended field dose and visual estimation of effects on 0 to 8 (see Table 1). However, 208 to 316 degree days (14 to 21 days) old plants showed a homogeneous phytochemical pattern 61 to 119 degree days (4 to 8 days) after exposure. 316 to 603 degree days (21 to 35 days) old plants showed a homogeneous phytochemical pattern 119 to 252 degree days (8 to 17 days) after exposure.

### Comparison of greenhouse and out-door grown plants

The comparison of a phytochemical pattern in plants grown in greenhouse and out-door is based on two greenhouse studies and one outdoor study. The two plant species A. *arvensis* and *Lólium perénne* were used to represent both a mono- and a dicotyledon species. In Table 3 the results of these studies are presented. In this table the comparison is based on similar ages 226 to 670 degree days (30 to 77 days) before exposure and the plants were harvested 180 to 240 degree days (14 to 17 days) after exposure. The greenhouse plants were more sensitive than the plants cultivated outdoors. New phytochemical compounds were present at 1 to 10% of recommended field dose for both plant species for plants grown in greenhouse. Almost the same pattern was seen for plants cultivated outdoor (see Table 3). In this case, the outdoor plants did not show new phytochemical compounds at 1 and 10% of recommended field dose, but the characteristic pattern was found at 50% of the recommended field dose. The phytochemical changes in outdoor grown plants might be found in lower concentrations. The visual effect seen on these plants varied from no effect to 5 (1 and 10% of recommended field dose of Roundup Bio) for greenhouse plants to 3 to 8 (see Table 1) for outdoor grown plants. This means that the phytochemical changes did not appear at low dosages in the field and the visual effects in the greenhouse plants and the field were similar.

For both plants species grown in greenhouse and outdoor, the pattern of new phytochemical compounds in plants exposed to Roundup Bio glyphosate varied with the age of the plants after exposure. Compounds appeared after 4 days and other dis-appered after 32 days, but a basic pattern was seen for all ages after exposure (see Table 4).For all plant species, the highest number of phytochemical changes was seen between 119 and 267 degree days (8 and 17 days) after exposure to glyphosate (Roundup Bio) in both greenhouse and outdoor grown plants.

### Discussion

Several studies have shown that herbicides cause phytochemical changes in plants. Earlier studies have focused on the presence and content of one or a few specific compounds, and no clear dose-response relationships have been established. In the present study the herbicide glyphosate was used seeking a systematic pattern of biochemical responses in plant species from important taxonomic groups. The use of TLC analysis includes a large variety of phytochemical compounds and allows for a broad screening. Despite around more than 2793 bioactive compounds from plants (Harbome and Baxter, 1995), relatively few compounds are expressed in plants and detected in the TLC-Systems. In general, the analysis suggests that specific patterns of phytochemical changes can be obtained using simple direct analysis of plant sap. This procedure could even be performed on-site in the field. The response differs between plant species, and three classes of sensitivity could be identified. Following exposure to glyphosate, a very specific pattern can be obtained by using different TLC-systems. Preliminary analysis of plants exposed to herbicides of different mode of action (i.e. pendimethalin, bromoxynil and metsulfuron methyl) show different patterns, allowing for qualitative identification of exposure to one or more herbicides. Other ongoing experiments show that natural stressors such as temperature and drought may change the concentration of compounds in the plant sap. After exposure to glyphosate, the phytochemical pattern showed minor differences when plants were developed either in greenhouse or outdoors.

The changed phytochemical pattern appeared in the annual plants about four days after exposure. The pattern was most stable in older plants, staying until scenescence. In younger plants a high mortality was observed, even at doses at or lower than 1% field dose. Apparently, only few species will survive such exposure the first weeks after emergence, while older plants are less affected. In most cases the phytochemical changes at concentrations of glyphosate, which did not cause any visual effects on the plants, were observed. By selecting sensitive plants species and optimising the choice of TLC system the pattern of phytochemical changes can be used as a sensitive and selective biomarker for glyphosate exposure. This unique pattern of biomarkers can easily be detected in vascular plants and evaluated by comparison with untreated controls.

**Table 2: Sensitivity of plants exposed to glyphosate (Roundup Bio), based on presence of phytochemical changes in all the TLC-Systems at lowest dose of concentration (LC) of the herbicide in per cent of field dose. The corresponding visual effect (VE) is presented (see table 1).**

| **Group I: Very sensitive plant species/families** | | **LC** | **VE** |
|---|---|---|---|
| *Anagallis arvensis* | Primuláceae | 1 | 0 |
| *Lólium perénne* | Poáceae | 1 | 0 |
| *Rumex crispus* | Polygonaceae | 1 | 0 to 1 |
| *Fallopia convolvulus* | Polygonaceae | 1 | 0 to 1 |
| *Rudbeckia hirta* | Asteráceae | 1 | 1 |
| *Plantágo lanceoláta* | Plantagináceae | 1 | 0 to 4 |
| *Papaver rhoeas* | Papaveraceae | 1 | 4 |

| **Group II: Medium sensitive plant species/families** | | **LC** | **VE** |
|---|---|---|---|
| *Centauréa cýanus* | Asteráceae | 1 to 10 | 0 to 3 |
| *Inula helenium* | Asteráceae | 1 to 10 | 0 to 5 |
| *Prunella vulgaris* | Laminaceae | 1 to 10 | 4 to 6 |
| *Digitalis lanceolata* | Scrophulariaceae | 1 to 10 | 0 to 5 |

| **Group III: Less sensitive plant species/families** | | | |
|---|---|---|---|
| *Leonorus carica* | Laminaceae | 10 | 5 |
| *Mentha spicata* | Laminaceae | 10 | 6 |
| *Nepeta cataria* | Laminaceae | 10 | 7 |
| *Sinapis avensis* | Brassicaceae | 10 | 7 |
| *Bellis perennis* | Asteraceae | 10 | 7 |

| Table 3: Comparison of phytochemical changes in Anagallis arvensis and Lólium perénne grown in greenhouse and outdoor after exposure to glyphosate (Roundup Bio). Mean Rf-value and colour of spots are shown for all five TLC-Systems. | | | | | | | |
|---|---|---|---|---|---|---|---|
| | | | | | | | |

| non-specific compounds (TLC - 7) | | | | | | | |
|---|---|---|---|---|---|---|---|
| | | A. arvensis | | | L. perenne | | |
| Colour | Mean Rf-value | Study 1 | Study 2 | Study 3 | Study 1 | Study 2 | Study 3 |
| Green | 0.01 | | | | x | x | |
| Grey/green* | 0.08 | x | x | x | x | x | |
| Blue/green | 0.29 | x | x | x | | x | |
| Red violet | 0.41 | x | x | x | | x | x |
| Blue/grey/green | 0.45 | x | x | x | | | |
| Green/red/yellow* | 0.66 | x | x | x | x | x | x |
| Grey/green | 0.83 | x | x | x | | | x |
| | | | | | | | |

| non-specific compounds (TLC - 1) | | | | | | | |
|---|---|---|---|---|---|---|---|
| | | A. arvensis | | | L. perenne | | |
| Colour | Mean Rf-value | Study 1 | Study 2 | Study 3 | Study 1 | study 2 | Study 3 |
| Blue green/red violet* | 0.04 | x | x | x | x | x | |
| Blue green* | 0.13 | | | | x | x | x |
| Blue green | 0.38 | x | x | x | x | x | |
| Blue | 0.44 | x | x | x | x | x | |
| Yellow | 0.5 | | | | x | | x |
| Blue | 0.63 | x | x | x | | | |
| Blue green/red violet | 0.76 | x | x | x | | | |
| Blue green | 0.94 | | | | x | x | x |
| | | | | | | | |

| Amino acids (TLC - 4d) | | A. arvensis | | | L. perenne | | |
|---|---|---|---|---|---|---|---|
| Colour | Mean Rf-value | Study 1 | Study 2 | Study 3 | Study 1 | Study 2 | Study 3 |
| Violet | 0.13 | X | x | x | x | x | |
| Red | 0.19 | X | x | x | x | x | x |
| Yellow | 0.45 | X | x | x | x | x | x |
| Violet* | 0.47 | X | x | x | x | x | x |
| Violet | 0.52 | | | | x | x | |
| Red | 0.51 | X | x | | | x | x |
| Violet* | 0.69 | X | x | x | x | x | x |
| Red* | 0.73 | X | x | x | x | x | x |
| | | | | | | | |

| Phenolic compounds (TLC - 2) | | | | | | | |
|---|---|---|---|---|---|---|---|
| | | A. arvensis | | | L. perenne | | |
| Colour (UV-light 366 nm) | Mean Rf-value | Study 1 | Study 2 | Study 3 | Study 1 | Study 2 | Study 3 |
| Blue* | 0.07 | X | x | | | | |
| Blue | 0.17 | | | | x | x | |
| Blue* | 0.5 | X | x | x | x | x | |
| Blue* | 0.62 | X | x | x | x | x | x |
| Blue | 0.74 | X | x | | x | | |
| Blue | 0.87 | X | x | | | | |
| Blue* | 0.92 | X | x | x | | x | x |
| Orange | 0.96 | | | | | x | |
| | | | | | | | |

| Lipids/terpens (TLC - 6) | | | | | | | |
|---|---|---|---|---|---|---|---|
| | | A. arvensis | | | L. perenne | | |
| Colour | Mean Rf-value | Study 1 | Study 2 | Study 3 | Study 1 | Study 2 | Study 3 |
| Blue* | 0.09 | X | x | x | x | x | |
| Blue | 0.21 | | | | x | x | |
| Blue | 0.32 | X | x | | | | |
| Blue | 0.46 | | | | | x | |
| Blue | 0.53 | X | x | x | x | x | x |
| Blue | 0.87 | X | x | x | | x | x |
| Study 1: Greenhouse; Plant age: 226-338 degree days (30 days) at exposure, harvested 180 degree days (14 days) after exposure | | | | | | | |
| Study 2: Greenhouse; Plant age: 891 degree days (39 days) at exposure, harvested 240 degree days (16 days) after exposure | | | | | | | |
| Study 3: Outdoor; Plant age: 670 degree days (77 days) at exposure, harvested 139 degree days (17 days) after exposure | | | | | | | |
| * Most frequent phytochemical changes found in more than 50% of all tested plant species in TLC 1, 2, 4d and 7, and in more than 81% of all tested plants in TLC 6. | | | | | | | |

**Table 4: Pattern of phytochemical changes in Anagallis arvensis and Lólium perénne grown in greenhouse and outdoors in relation to days after exposure with Roundup Bio. Rf-values and colour of spots for TLC-System 4d representing an amino acid pattern.**

| *A. arvensis* grown in greenhouse | | | | | |
|---|---|---|---|---|---|
| Amino acids (TLC 4d) | | | | | |
| Colour | Mean Rf-value | 60 dd (4 days) | 120 dd (8 days) | 240 dd (16 days) | 480 dd (32 days) |
| Violet | 0.12 | | x | x | X |
| Red | 0.16 | | x | x | |
| Yellow | 0.39 | x | x | x | x |
| Violet | 0.49 | x | x | x | x |
| Red | 0.56 | | x | x | |
| Violet | 0.65 | x | x | x | x |
| Red | 0.73 | x | x | x | x |
| | | | | | |

| *A. arvensis* grown outdoor | | | | | |
|---|---|---|---|---|---|
| Amino acids (TLC 4d) | | | | | |
| Colour | Mean Rf-value | 32 dd (4 days) | 61 dd (8 days) | 139 dd (16 days) | 242 dd (27 days) |
| Violet | 0.12 | | x | x | x |
| Red | 0.16 | | x | x | x |
| Yellow | 0.39 | x | x | x | x |
| Violet | 0.49 | x | x | x | x |
| Red | 0.56 | x | | | |
| Violet | 0.65 | x | x | x | x |
| Red | 0.73 | x | x | x | x |
| | | | | | |

| *L. perenne* grown in greenhouse | | | | | |
|---|---|---|---|---|---|
| Amino acids (TLC 4d) | | | | | |
| Colour | Mean Rf-value | 60 dd (4 days) | 120 dd (8 days) | 240 dd (16 days) | 480 dd (32 days) |
| Violet | 0.12 | x | x | x | x |
| Red | 0.16 | | x | x | x |
| Violet | 0.24 | | x | | |
| Yellow | 0.36 | | x | x | x |
| Violet | 0.42 | | x | x | |
| Red | 0.51 | | | x | |
| Violet | 0.53 | | x | x | x |
| Red | 0.58 | | x | x | x |
| Violet | 0.64 | x | x | x | x |
| Red | 0.73 | | x | x | x |
| | | | | | |

| *L. perenne* grown outdoor | | | | | |
|---|---|---|---|---|---|
| Amino acids (TLC 4d) | | | | | |
| Colour | Mean Rf-value | 32 dd (4 d) | 61 dd (7 days) | 139 dd (17 days) | 242 dd (27 days) |
| Red | 0.16 | n.d. | | x | |
| Yellow | 0.36 | n.d. | | x | x |
| Violet | 0.42 | n.d. | x | x | x |
| Violet | 0.53 | n.d. | | x | x |
| Red | 0.58 | n.d. | | | x |
| Violet | 0.64 | n.d. | | | x |
| Red | 0.73 | n.d. | | | x |

| | | | | | |
|---|---|---|---|---|---|
| d: Days after exposure; dd: degree days after exposure | | | | | |

## Claims

1. A method for testing whether material from a specific plant has been exposed to a specific herbicide, comprising the steps of:
- obtaining material from the specific plant,
- providing an assayable form of at least a part of said plant material,
- detecting by thin layer chromatography a pattern of a biomarker composition of said assayable form of at least two biomarkers, said biomarkers being phytochemical compounds selected from the group of amino acids, sugars, glycosides and N-containing compounds, which after the plant has been exposed to said herbicide, either increase or decrease in concentration, are eliminated, or are newly produced, and
- correlating said pattern of a biomarker composition to a standard biomarker pattern obtained by thin layer chromatography for said specific herbicide and said specific plant,
- assessing whether said plant has been exposed to said specific herbicide.

2. The method according to claim 1, wherein said standard biomarker pattern is produced by:
- subjecting said specific plant to said specific herbicide,
- obtaining material from said specific plant,
- determining the phytochemical responses of said material from said specific plant for said specific herbicide, and
- hereby obtaining at least one standard biomarker pattern relating to said specific herbicide.

3. The method of testing according to claim 1 or 2, wherein said pattern of a biomarker composition is also correlated to a standard pattern, wherein said standard pattern is a pattern of the composition of compounds present in a plant before exposure to any herbicide.

4. The method of testing according to claim 1-3, wherein the herbicide is selected from a group consisting of Glyphosate, Bromoxynil, Pendimethalin and Metsulfuron methyl.

5. The method of testing according to any of the preceding claims, wherein the plant material is fresh.

6. The method of testing according to any of the preceding claims, wherein the plant material is frozen.

7. The method of testing according to any of the preceding claims, wherein the plant material is dry.

8. The method of testing according to claim 7, wherein the plant material is air dried.

9. The method of testing according to claim 7, wherein the plant material is dried by nitrogen gas.

10. The method of testing according to claim 7, wherein the plant material is freeze dried.

11. The method of testing according to claim 7, wherein the plant material is dried by fluid nitrogen.

12. The method of testing according to claim 7, wherein the plant material is heat dried.

13. The method of testing according to claim 7, wherein the plant material is sun dried.

14. The method of testing according to any of the preceding claims, wherein the plant material is selected from at least part of a flower.

15. The method of testing according to any of the preceding claims, wherein the plant material is selected from at least part of a shoot.

16. The method of testing according to any of the preceding claims, wherein the plant material is selected from at least part of a leaf.

17. The method of testing according to any of the preceding claims, wherein the plant material is selected from at least part of a stem.

18. The method of testing according to any of the preceding claims, wherein the plant material is selected from at least part of a root.

19. The method of testing according to any of the preceding claims, wherein the plant material is selected from at least part of a seed.

20. The method of testing according to any of the preceding claims, wherein the pattern of the composition of more than 3 biomarkers is determined.

21. The method of testing according to any of the preceding claims, wherein the assessment is qualitative and/or quantitative and/or semi-quantitative.

22. The method of testing according to any of the preceding claims, wherein the use of Thin Layer Chromatography comprises the following steps:
- contacting an assayable form of said plant material with a TLC-plate,
- subjecting said TLC-plate to a solvent, such as an eluent,
- obtaining a biomarker pattern.

23. The method according to claim 22, further comprising the following steps before obtaining a biomarker pattern, drying said TLC-plate and/or contacting said TLC-plate with a chemical reagent.

24. The method according to claim 22 or 23, wherein the solvent comprises n-butanol and formic acid.

25. The method of testing according to the claims 1-24, comprising the following steps:
- contacting the assayable form of said plant material with a support for receiving said plant material,
- subjecting said support to a solvent,
- drying said support,
- obtaining a biomarker pattern.

26. The method of testing according to claim 25, further comprising the step of contacting said support with a chemical reagent before obtaining a biomarker pattern.

27. The method of testing according to any of the preceding claims, wherein the pattern of the biomarker composition is obtained as a result of the specific combination of parameters, such as said support, said solvent and said chemical reagent.

28. Use of a method of testing as defined in the claims 1-27 in food quality control.

29. Use of a method of testing as defined in the claims 1-27 for the control of crops.

30. Use of a method of testing as defined in the claims 1-27 for the control of weeds.

31. Use of a method of testing as defined in the claims 1-27 for the control of whether organic crop has been exposed to a herbicide.

32. Use of a method of testing as defined in the claims 1-27 in control of gene modified plants.

33. Use of a method of testing as defined in the claims 1-27 in control of the geographic distribution of pesticides.

34. Use of a method of testing as defined in the claims 1-27 in effect studies of pesticides.

## Patentansprüche

1. Verfahren zum Testen, ob Material aus einer spezifischen Pflanze einem spezifischen Herbizid ausgesetzt gewesen ist, umfassend die Schritte:
- Erhalten von Material von der spezifischen Pflanze,
- Bereitstellen einer untersuchbaren Form von wenigstens einem Teil des Pflanzenmaterials,
- Detektieren durch Dünnschichtchromatographie eines Musters einer Biomarkerzusammensetzung der untersuchbaren Form von wenigstens zwei Biomarkern, wobei die Biomarker phytochemische Verbindungen sind, die aus der Gruppe aus Aminosäuren, Zuckern, Glykosiden und N-haltigen Verbindungen ausgewählt sind, die, nachdem die Pflanze dem Herbizid ausgesetzt gewesen ist, in ihrer Konzentration entweder ansteigen oder abnehmen, eliminiert oder neu produziert werden, und
- Korrelieren des Biomarkerzusammensetzungsmusters mit einem Standardbiomarkermuster, das durch Dünnschichtchromatographie für das spezifische Herbizid und die spezifische Pflanze erhalten ist,
- Abschätzen, ob die Pflanze dem spezifischen Herbizid ausgesetzt gewesen ist.

2. Verfahren nach Anspruch 1, wobei das Standardbiomarkermuster erhalten wird durch:
- Aussetzen der spezifischen Pflanze dem spezifischen Herbizid,
- Erhalten von Material von der spezifischen Pflanze,
- Bestimmen der phytochemischen Antworten des Materials aus der spezifischen Pflanze für das spezifische Herbizid und
- dadurch Erhalten wenigstens ein Standardbiomarkermuster betreffend das spezifische Herbizid.

3. Verfahren zum Testen nach Anspruch 1 oder 2, wobei das Muster einer Biomarkerzusammensetzung auch mit einem Standardmuster korreliert, wobei das Standardmuster ein Muster der Zusammensetzung von Verbindungen ist, die in einer Pflanze vorhanden sind, bevor sie irgendeinem Herbizid ausgesetzt wird.

4. Verfahren zum Testen nach einem der Ansprüche 1 bis 3, wobei das Herbizid aus einer Gruppe bestehend aus Glyphosat, Bromoxynil, Pendimethanil und Metsulfuronmethyl ausgewählt ist.

5. Verfahren zum Testen nach einem der vorhergehenden Ansprüche, wobei das Pflanzenmaterial frisch ist.

6. Verfahren zum Testen nach einem der vorhergehenden Ansprüche, wobei das Pflanzenmaterial gefroren ist.

7. Verfahren zum Testen nach einem der vorhergehenden Ansprüche, wobei das Pflanzenmaterial trocken ist.

8. Verfahren zum Testen nach Anspruch 7, wobei das Pflanzenmaterial luftgetrocknet ist.

9. Verfahren zum Testen nach Anspruch 7, wobei das Pflanzenmaterial durch Stickstoffgas getrocknet ist.

10. Verfahren zum Testen nach Anspruch 7, wobei das Pflanzenmaterial gefriergetrocknet ist.

11. Verfahren zum Testen nach Anspruch 7, wobei das Pflanzenmaterial durch flüssigen Stickstoff getrocknet ist.

12. Verfahren zum Testen nach Anspruch 7, wobei das Pflanzenmaterial hitzegetrocknet ist.

13. Verfahren zum Testen nach Anspruch 7, wobei das Pflanzenmaterial sonnengetrocknet ist.

14. Verfahren zum Testen nach einem der vorhergehenden Ansprüche, wobei das Pflanzenmaterial von wenigstens einem Teil einer Blume ausgewählt ist.

15. Verfahren zum Testen nach einem der vorhergehenden Ansprüche, wobei das Pflanzenmaterial von wenigstens einem Teil eines Schößlings ausgewählt ist.

16. Verfahren zum Testen nach einem der vorhergehenden Ansprüche, wobei das Pflanzenmaterial aus wenigstens einem Teil eines Blattes ausgewählt ist.

17. Verfahren zum Testen nach einem der vorhergehenden Ansprüche, wobei das Pflanzenmaterial aus wenigstens einem Teil eines Stammes ausgewählt ist.

18. Verfahren zum Testen nach einem der vorhergehenden Ansprüche, wobei das Pflanzenmaterial aus wenigstens einem Teil einer Wurzel ausgewählt ist.

19. Verfahren zum Testen nach einem der vorhergehenden Ansprüche, wobei das Pflanzenmaterial aus wenigstens einem Teil eines Samens ausgewählt ist.

20. Verfahren zum Testen nach einem der vorhergehenden Ansprüche, wobei das Muster der Zusammensetzung aus mehr als drei Biomarkern bestimmt ist.

21. Verfahren zum Testen nach einem der vorhergehenden Ansprüche, wobei die Abschätzung qualitativ und/oder quantitativ und/oder semiquantitativ ist.

22. Verfahren zum Testen nach einem der vorhergehenden Ansprüche, wobei die Verwendung der Dünnschichtchromatographie die folgenden Schritte umfaßt:
- Berühren einer untersuchbaren Form des Pflanzenmaterials mit einer TLC-Platte,
- Aussetzen der TLC-Platte einem Lösungsmittel, wie z.B. ein Elutionsmittel,
- Erhalten eines Biomarkermusters.

23. Verfahren nach Anspruch 22, ferner umfassend die folgenden Schritte vor Erhalten eines Biomarkermusters, Trocknen der TLC-Platte und/oder Berühren der TLC-Platte mit einem chemischen Reagenz.

24. Verfahren nach Anspruch 22 oder 23, wobei das Lösungsmittel n-Butanol und Ameisensäure umfaßt.

25. Verfahren zum Testen nach einem der Ansprüche 1 bis 24, umfassend die folgenden Schritte:
- Berühren der untersuchbaren Form des Pflanzenmaterials mit einem Träger zum Aufnehmen des Pflanzenmaterials,
- Aussetzen des Trägers einem Lösungsmittel,
- Trocknen des Trägers,
- Erhalten eines Biomarkermusters.

26. Verfahren zum Testen nach Anspruch 25, ferner umfassend den Schritt des Berührens des Trägers mit einem chemischen Reagenz vor dem Erhalten eines Biomarkermusters.

27. Verfahren zum Testen nach einem der vorhergehenden Ansprüche, wobei das Muster der Biomarkerzusammensetzung als ein Ergebnis der spezifischen Kombination von Parametern erhalten ist, wie z.B. dem Träger, dem Lösungsmittel und dem chemischen Reagenz.

28. Verwendung eines Verfahrens zum Testen nach einem der Ansprüche 1 bis 27 in der Lebensmittelqualitätskontrolle.

29. Verwendung eines Verfahrens zum Testen nach einem der Ansprüchen 1 bis 27 zur Kontrolle von Erntefrüchten.

30. Verwendung eines Verfahrens zum Testen nach einem der Ansprüche 1 bis 27 zur Kontrolle von Unkräutern.

31. Verwendung eines Verfahrens zum Testen nach einem der Ansprüche 1 bis 27 zur Kontrolle, ob Bioerntefrüchte einem Herbizid ausgesetzt gewesen sind.

32. Verwendung eines Verfahrens zum Testen nach einem der Ansprüche 1 bis 27 in der Kontrolle von genmodifizierten Pflanzen.

33. Verwendung eines Verfahrens zum Testen nach einem der Ansprüche 1 bis 27 in der Kontrolle der geographischen Verteilung von Pestiziden.

34. Verwendung eines Verfahrens zum Testen nach einem der Ansprüche 1 bis 27 in Wirksamkeitsstudien von Pestiziden.

## Revendications

1. Procédé d'analyse permettant de déterminer si un matériel provenant d'une plante spécifique a été exposé à un herbicide spécifique, comprenant les étapes suivantes :
- l'obtention d'un matériel provenant de la plante spécifique,
- la fourniture d'une forme analysable d'au moins une partie dudit matériel végétal,
- la détection par chromatographie sur couche mince d'un profil d'une composition de biomarqueurs de ladite forme analysable d'au moins deux biomarqueurs, lesdits biomarqueurs étant des composés phytochimiques choisis dans le groupe des acides aminés, des sucres, des glucosides et des composés azotés, qui voient leur concentration augmenter ou diminuer, sont éliminés ou sont nouvellement produits après l'exposition de la plante audit herbicide, et
- la corrélation dudit profil d'une composition de biomarqueurs avec un profil de biomarqueurs étalon obtenu par chromatographie sur couche mince pour ledit herbicide spécifique et ladite plante spécifique,
- le fait de déterminer si ladite plante a été exposée audit herbicide spécifique.

2. Procédé selon la revendication 1, dans lequel ledit profil de biomarqueurs étalon est produit par :
- la soumission de ladite plante spécifique audit herbicide spécifique,
- l'obtention d'un matériel provenant de ladite plante spécifique,
- la détermination des réponses phytochimiques dudit matériel provenant de ladite plante spécifique pour ledit herbicide spécifique, et
- l'obtention de cette manière d'au moins un profil de biomarqueurs étalon relatif audit herbicide spécifique.

3. Procédé d'analyse selon la revendication 1 ou 2, dans lequel ledit profil d'une composition de biomarqueurs est également corrélé avec un profil étalon, dans lequel ledit profil étalon est un profil de la composition de composés présents dans une plante avant son exposition à un quelconque herbicide.

4. Procédé d'analyse selon l'une quelconque des revendications 1 à 3, dans lequel l'herbicide est choisi dans le groupe constitué du glyphosate, du bromoxynil, de la pendiméthaline et du metsulfuron méthyl.

5. Procédé d'analyse selon l'une quelconque des revendications précédentes, dans lequel le matériel végétal est frais.

6. Procédé d'analyse selon l'une quelconque des revendications précédentes, dans lequel le matériel végétal est congelé.

7. Procédé d'analyse selon l'une quelconque des revendications précédentes, dans lequel le matériel végétal est sec.

8. Procédé d'analyse selon la revendication 7, dans lequel le matériel végétal est séché à l'air.

9. Procédé d'analyse selon la revendication 7, dans lequel le matériel végétal est séché avec de l'azote gazeux.

10. Procédé d'analyse selon la revendication 7, dans lequel le matériel végétal est lyophilisé.

11. Procédé d'analyse selon la revendication 7, dans lequel le matériel végétal est séché avec de l'azote fluide.

12. Procédé d'analyse selon la revendication 7, dans lequel le matériel végétal est séché par de la chaleur.

13. Procédé d'analyse selon la revendication 7, dans lequel le matériel végétal est séché au soleil.

14. Procédé d'analyse selon l'une quelconque des revendications précédentes, dans lequel le matériel végétal est choisi parmi au moins une partie d'une fleur.

15. Procédé d'analyse selon l'une quelconque des revendications précédentes, dans lequel le matériel végétal est choisi parmi au moins une partie d'une pousse.

16. Procédé d'analyse selon l'une quelconque des revendications précédentes, dans lequel le matériel végétal est choisi parmi au moins une partie d'une feuille.

17. Procédé d'analyse selon l'une quelconque des revendications précédentes, dans lequel le matériel végétal est choisi parmi au moins une partie d'une tige.

18. Procédé d'analyse selon l'une quelconque des revendications précédentes, dans lequel le matériel végétal est choisi parmi au moins une partie d'une racine.

19. Procédé d'analyse selon l'une quelconque des revendications précédentes, dans lequel le matériel végétal est choisi parmi au moins une partie d'une graine.

20. Procédé d'analyse selon l'une quelconque des revendications précédentes, dans lequel le profil de la composition de plus de 3 biomarqueurs est déterminé.

21. Procédé d'analyse selon l'une quelconque des revendications précédentes, dans lequel la détermination est qualitative et/ou quantitative et/ou semi-quantitative.

22. Procédé d'analyse selon l'une quelconque des revendications précédentes, dans lequel l'utilisation de la chromatographie sur couche mince comprend les étapes suivantes :
- le contact d'une forme analysable dudit matériel végétal avec une plaque CCM,
- la soumission de ladite plaque CCM à un solvant, tel qu'un éluant,
- l'obtention d'un profil de biomarqueurs.

23. Procédé selon la revendication 22, comprenant en outre les étapes suivantes avant l'obtention d'un profil de biomarqueurs, le séchage de ladite plaque CCM et/ou le contact de ladite plaque CCM avec un réactif chimique.

24. Procédé selon la revendication 22 ou 23, dans lequel le solvant comprend du n-méthanol et de l'acide formique.

25. Procédé d'analyse selon l'une quelconque des revendications 1 à 24, comprenant les étapes suivante :
- le contact de la forme analysable dudit matériel végétal avec un support pour recevoir ledit matériel végétal,
- la soumission dudit support à un solvant,
- le séchage dudit support,
- l'obtention d'un profil de biomarqueurs.

26. Procédé d'analyse selon la revendication 25, comprenant en outre l'étape de contact dudit support avec un réactif chimique avant l'obtention d'un profil de biomarqueurs.

27. Procédé d'analyse selon l'une quelconque des revendications précédentes, dans lequel le profil de la composition de biomarqueurs est obtenu du fait de la combinaison spécifique de paramètres, tels que ledit support, ledit solvant et ledit réactif chimique.

28. Utilisation d'un procédé d'analyse tel que défini dans les revendications 1 à 27 pour contrôler la qualité d'un aliment.

29. Utilisation d'un procédé d'analyse tel que défini dans les revendications 1 à 27 pour contrôler les cultures.

30. Utilisation d'un procédé d'analyse tel que défini dans les revendications 1 à 27 pour lutter contre les mauvaises herbes.

31. Utilisation d'un procédé d'analyse tel que défini dans les revendications 1 à 27 pour vérifier qu'une culture organique a été exposée à un herbicide.

32. Utilisation d'un procédé d'analyse tel que défini dans les revendications 1 à 27 pour contrôler les plantes génétiquement modifiées.

33. Utilisation d'un procédé d'analyse tel que défini dans les revendications 1 à 27 pour contrôler la répartition géographique des pesticides.

34. Utilisation d'un procédé d'analyse tel que défini dans les revendications 1 à 27 pour étudier les effets des pesticides.
